# FASCICULE DE BREVET EUROPEEN

(11) **EP 0 661 279 B1**
(45) Date de publication et mention de la délivrance du brevet: **07.03.2001**
(21) Numéro de dépôt: 94403038.6
(22) Date de dépôt: 27.12.1994
(51) Int. Cl.: C07D 257/02, C07C 229/16, A61K 49/00, C07H 15/26

(54) **Ligands polyaminés, complexes métalliques, procédé de préparation, applications diagnostiques et thérapeutiques**
Polyaminierte Liganden, Metallkomplexe, Verfahren zur Herstellung und diagnostische und therapeutische Verwendungen
Polyaminated ligands, metal complexes, process for their preparation and diagnostic and therapeutic applications

(30) Priorité: 30.12.1993 FR 9315933
(43) Date de publication de la demande: 05.07.1995
(73) Titulaire: GUERBET, 93420 Villepinte (FR)
(72) Inventeur: Meyer, Dominique, F-94100 Saint-Maur (FR); Rousseaux, Olivier, F-60300 Senlis (FR); Schaeffer, Michel, F-77400 Lagny (FR); Simonot, Christian, F-75020 Paris (FR)
(74) Mandataire: Le Guen, Gérard

(56) Documents cités:
- EP-A- 0 382 583
- EP-A- 0 484 989
- EP-A- 0 529 645
- EP-A- 0 565 930
- WO-A-89/12631

## Description

L'invention concerne des dérivés de poly(aminoacides) pouvant former des chélates avec les cations métalliques paramagnétiques, ainsi que ces chélates, les procédés de préparation de ces composés et les compositions pour l'imagerie médicale qui contiennent ces chélates.

En effet, ces complexes métalliques modifient les temps de relaxation des protons, excités par une radio-fréquence, dans un champ magnétique, et sont particulièrement utiles comme agents de contraste in vivo pour améliorer les images des organes cibles obtenues par résonance magnétique nucléaire.

Les complexes de gadolinium, utilisés en clinique humaine, correspondent à une relaxivité spin-réseau, ou longitudinale, R₁ comprise entre 3 et 5 mM⁻¹s⁻¹ dans l'eau, à 37°C pour 20 MHz.

Afin d'améliorer la qualité des images, on assiste actuellement à une augmentation des doses, par exemple 0,3 mmole/kg de poids corporel au lieu de 0,1 mmole/kg pour les complexes classiques comportant un seul ion Gd par molécule, augmentation qui peut s'accompagner d'un accroissement des effets secondaires, notamment ceux dus à l'osmolalité des complexes.

Il serait évidemment préférable d'augmenter l'intensité du signal mesuré en augmentant la relaxivité R₁ de l'agent de contraste.

On sait que R₁ est augmenté sensiblement lorsque le chélate métallique est greffé sur une macromolécule d'origine biologique ou non, tel que dextran, albumine ou polylysine; néanmoins si la relaxivité R₁ par atome de gadolinium augmente, le rapport de R₁ à la masse moléculaire du complexe conjugué, dit relaxivité massique, diminue de telle sorte que le poids d'une dose diagnostique augmente, ainsi que son coût.

Les complexes de gadolinium de l'invention donnent des relaxivités R₁ supérieures à celles des complexes connus de masses moléculaires analogues; il est vraisemblable mais on ne saurait être limité par cette explication, que l'introduction d'au moins 3 bras latéraux hydrophiles sur les groupes acides, substituants les atomes d'azote donneurs des ligands connus, diminue sensiblement la liberté de mouvement du complexe paramagnétique et de l'ion paramagnétique qui y est attaché, dont la rotation dans le champ magnétique est ainsi restreinte.

La présence de bras latéraux a parfois été envisagée dans certaines demandes de brevet, telles que EP-A-299795, EP-A-481420 et WO 89/05802, mais seulement à titre de généralisation de formules, exemplifiées uniquement par des molécules dont les ramifications sont courtes, plus ou moins hydrophobes et situées sur deux des azotes au plus, de telle sorte qu'aucun effet favorable ne pouvait être observé sur la relaxivité massique et n'a, évidemment, été envisagé.

Par un choix correct des bras latéraux, caractéristiques de l'invention, on peut, et c'est un autre avantage, non seulement améliorer la relaxivité du complexe mais aussi agir sur sa biodistribution, par exemple en introduisant dans ces bras des fragments spécifiques de certains récepteurs biologiques ou encore en utilisant des bras de dimension telle que le volume moléculaire du complexe soit suffisant pour diminuer sa perméabilité vasculaire et qu'il séjourne dans cette zone plus longtemps que les agents de contraste actuels.

Selon un premier aspect, l'invention concerne des composés de type poly(aminoacides), qui peuvent former des chélates avec les ions métalliques paramagnétiques, caractérisés en ce qu'au moins 3 des atomes d'azote donneurs, à savoir ceux qui formeront des liaisons de coordination avec l'ion métallique, portent des substituants, identiques ou différents, de formule CH(R₁)-X, dans laquelle X représente CO₂Rₐ, CONR_{b}R_{c} ou P(R_{d})O₂H et Rₐ, R_{b}, R_{c} représentent indépendamment H ou (C₁-C₈)alkyle, éventuellement hydroxylé, et R_{d} représente OH, (C₁-C₈)alkyle ou (C₁-C₈) alkoxy,
et R₁ représente un groupe hydrophile de masse moléculaire supérieure à 200, comportant au moins 3 atomes d'oxygène,
étant entendu qu'au moins 3 des groupes X sont des fonctions acides, éventuellement salifiées.

R₁ peut comporter des atomes d'azote, mais aucun ne saurait être un atome donneur du chélate, c'est-à-dire former une liaison de coordination avec l'ion métallique.

La plupart des ligands connus pour complexer les cations paramagnétiques tels que Fe³⁺, Mn²⁺, Gd³⁺, Dy³⁺, ou même les éléments radioactifs comme l'yttrium ou le technétium comportent au moins 3 atomes d'azote substitués par un groupe acétique, méthylènephosphonique ou phosphorique, mais les molécules de la présente invention s'en différencient par la présence sur ces 3 substituants d'un bras latéral fonctionnalisé hydrophile. Ces bras, ou ramifications, peuvent être de nature très diverse mais ils doivent présenter suffisamment d'encombrement et comporter des atomes susceptibles de former in vivo avec le milieu environnant des liaisons de telle sorte que ces interactions moléculaires immobilisent la molécule dans le milieu par au moins 3 points.

Ainsi, le remplacement dans les molécules connues pour complexer des ions métalliques paramagnétiques, des substituants d'au moins 2 des atomes d'azote donneurs par 3, ou mieux 4 groupes CH(R₁)-X conformes à l'invention, de préférence identiques, permet d'obtenir des composés selon l'invention.

On peut notamment citer parmi ces molécules connues, celles décrites dans EP-A-232 751, EP-A-255 471, EP-A-287 465, EP-A-365 412, EP-A-391 766, EP-A-438 206, EP-A-484 989, EP-A-499 501, WO-89/01476, WO-89/10645, et WO-91/11475 ainsi que le ligand du gadopentate et celui du gadotérate.

Parmi les groupes R₁ convenables, certains ne comportent que des atomes de C, H et O; ce sont notamment des poly[oxy(C₂-C₃)alkylènes], des polyhydroxyalkyles ou les restes d'oligosaccharides ou de polysaccharides, monofonctionnalisés pour permettre leur fixation sur le carbone en alpha de X.

R₁ peut aussi représenter des groupes plus complexes et notamment

R₂-G-R₃,

dans lequel
R₂ représente rien, alkylène, alkoxyalkylène, polyalkoxyalkylène, alkylène interrompu par phénylène, phénylène ou un reste hétérocyclique saturé ou non,
G représente une fonction O, CO, OCO, COO, SO₃, OSO₂, CONR', NR'CO, NR'COO, OCONR', NR', NR'CS, CSNR', SO₂NR', NR'SO₂, NR'CSO, OCSNR', NR'CSNR', P(O)(OH)NR', NR'P(O)-(OH) dans laquelle R' est H, (C₁-C₈)alkyle ou R₃
R₃ représente alkyle, phényle, alkyle substitué ou interrompu par un ou des groupes choisis parmi phényle, alkylèneoxy, amino ou amido substitués ou non par alkyle éventuellement substitué ou interrompu par l'un des groupes précédents ou R₃ est le reste d'un composé, éventuellement monofonctionnalisé, choisi parmi les saccharides, les oligosaccharides, les peptides, les macromolécules naturelles ou synthétiques biocompatibles ou les molécules susceptibles de se lier à un biorécepteur endogène,
ainsi que les sels de ces composés avec des acides ou des bases physiologiquement acceptables.

On préfère les composés dans lesquels G est un groupe amido : CONR' ou NR'CO, R' étant H,(C₁-C₈)alkyle ou R₃, ou est l'atome d'oxygène, formant avec R₂ et R₃ une fonction éther-oxyde ainsi que ceux dans lesquels X est CO₂H.

Parmi ceux-ci, les composés dans lesquels les R₁, identiques ou différents, représentent R₂-G-R₃ sont particulièrement préférés lorsque R₂ représente (C₀-C₆)alkylène, éventuellement interrompu par phénylène et R₃ représente (C₁-C₁₄)alkyle éventuellement substitué ou interrompu par un ou des groupes choisis parmi phényle, (C₁-C₆)alkoxy, amino et amido substitués ou non par alkyle ou alkoxyalkyle, les saccharides, les oligo-saccharides et les macromolécules biocompatibles telles que polyéthylèneglycol et ses éthers en C₁-C₂ et dextran.

Comme R₂ préféré, on peut citer (CH₂)ₙ, CH₂CHOH, CH₂CHOHCH₂, (CH₂)₄CHOH, (CH₂)ₙC₆H₄ ou C₆H₄ avec n = 1, 2, 3.

Dans l'ensemble de la présente description, sauf mention contraire, on entend par poly[oxy(C₂-C₃)alkylène], les polyoxyéthylènes et polyoxypropylènes notamment le polyéthylène glycol et ses monoéthers et monoesters en C₁ à C₃, de masse moléculaire inférieure à 150 000; par saccharides, on entend les hydrates de carbone, tel que mannose, fucose, galactose et les aminosaccharides tel que glucosamine ou galactosamine; par oligosaccharide, on entend les enchaînements linéaires ou cycliques de 2 à 10 unités saccharidiques, tel que le saccharose, le maltotriose et les cyclodextrines; par polysaccharides, on entend notamment les dérivés de la cellulose, ou l'hydroxyéthylamidon, l'inuline ou les dextrans de masse moléculaire inférieure à 20 000 ou même supérieure pour les complexes insolubles dans l'eau; par poly(hydroxyalkyle) on entend les polyols de masse moléculaire inférieure à 20 000 et notamment l'alcool polyvinylique.

Les groupes alkyles, alkylènes, alkoxys sont sauf mention contraire en C₁ à C₁₄, linéaires, ramifiés ou cycliques; ces groupes peuvent être hydroxylés sur un ou plusieurs carbones.

Les groupes phényles, phénylènes et hétérocycliques peuvent être substitués par OH, Cl, Br, I, (C₁-C₈)alkyle, (C₁-C₈)alkoxy, NO₂, NRₓR_{y}, NR_{X}COR_{y}, CONRₓR_{y}, COORₓ, Rₓ et R_{y} étant H ou (C₁-C₈)alkyle.

Parmi les groupes hétérocycliques, aromatiques, insaturés ou acycliques, on peut citer ceux dérivés du thiophène, furanne, pyranne, pyrrole, pyrrolidine, morpholine, piperazine, imidazole, pyridine, pyrimidine, pyrazine, pyridazine, thiazole, oxazole, pyrrolidine, imidazoline, dioxanne, tétrazole, benzofuranne, indole, quinoléine et isomères ou dérivés plus ou moins saturés.

Parmi les macromolécules naturelles ou synthétiques biocompatibles on peut citer les polyoxy(C₂-C₃)alkylène ou polyéthers, les polysaccharides, les polyaminoacides, comme la polylysine, les protéines comme l'albumine ou les anticorps et leurs fragments, les glycoprotéines ainsi que les oligomères ou polymères en étoile comme les dendrimères et les arborols décrits dans Angew. Chemie, Int. Ed. 29(2) 138-175, 1990 et EP-A-115 771.

On peut aussi, notamment lorsque les chélates sont destinés à être administrés à l'homme par voie orale ou rectale, utiliser des macromolécules insolubles ou peu solubles dans l'eau, telles que les dérivés des acides polyméthacryliques ou la polyvinylpyrrolidone.

Parmi les molécules susceptibles de se lier à un biorécepteur endogène, et permettant donc de concentrer le chélate dans un organe ou une partie de celui-ci on peut citer celles mentionnées dans US-4 647 447 et notamment les hormones comme l'insuline, les prostaglandines, les stéroïdes, les anticorps, notamment ceux spécifiques des cellules tumorales, les lipides ou certains sucres comme l'arabinogalactane ou le glucose, des glycoprotéines sans acide sialique terminal connus pour leur fixation hépatique.

Par ailleurs, la présence d'une zone hydrophobe sur R₁, et notamment celle d'un noyau phényle, peut favoriser ainsi la formation de liaisons non-covalentes avec les protéines biologiques et notamment avec l'albumine; cette zone hydrophobe peut aussi se trouver greffée sur une autre partie du poly(aminoacide).

Par (poly)saccharide monofonctionnalisé, on entend un (poly)saccharide dont l'une des unités saccharidiques à l'extrémité de la chaîne a été modifiée pour permettre la formation de la liaison G-R₃ ou CH-R₁; ce type de fonctionnalisation décrit notamment dans J. Polymer. Sc. Part A Polymer chemistry 23 1395-1405 (1985) et 29, 1271-1279 (1991) et dans Bioconjugate chem. 3, 154-159 (1992) est effectué par amination réductrice avec NH₃ ou une amine comportant un groupe réactif ou précurseur d'un groupe réactif ou par oxydation pour donner une lactone. On peut ainsi obtenir un dérivé ayant comme fonction terminale une amine primaire ou un dérivé portant un groupe réactif, comme 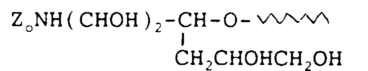 avec Zₒ = H,H₂N(CH₂)ₙ, O₂NC₆H₄CH₂ ou un dérivé comportant une fonction acide 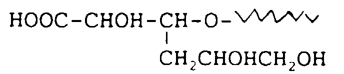 à partir du maltose
tandis que pour le dextran, on obtiendra

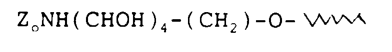

Par polyéthylèneglycol monofonctionnalisé ou par éther de polyéthylène glycol monofonctionnalisé, on entend le composé dont l'une des extrémités porte une fonction réactive, telle que celles décrites dans JMS Res. Macromol. Chem. Phys. C. 25(3) 325-373 (1985); on peut aussi se référer à J. Org. Chem. 45, 5364 (1980) pour la préparation d'un aminopolyéthylène-glycol ou à Makromol. Chem. 182, 1379-1384 (1981) pour différentes méthodes de substitution.

Un groupe préféré de ligands de l'invention est de formule 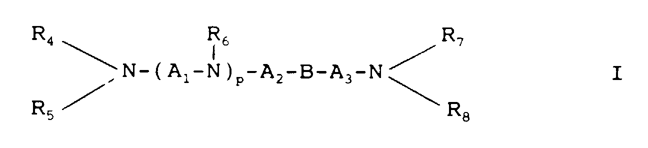 dans laquelle
- A₁, A₂, A₃, indépendamment l'un de l'autre, représentent 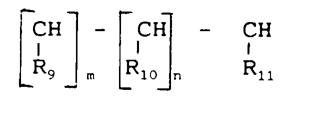 m et n étant des nombres entiers, identiques ou différents, compris entre 0 et 5 dont la somme vaut 1 à 5.
   R₉, R₁₀, R₁₁ représentent, indépendamment H, alkyle, alkoxyalkyle, phényle, alkylènephényle, R₁₀ pouvant représenter en outre OH ou alkoxy
   ou l'un des R₉ et R₁₁ parmi A₁, A₂, A₃ représente la formule 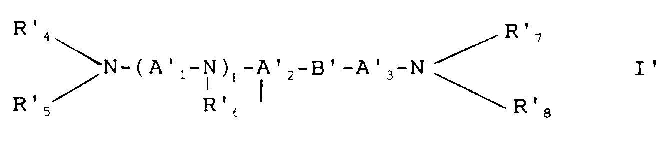 dans laquelle les lettres peuvent avoir les significations des lettres de même indice de la formule I, à l'exclusion de l'un des R'₉ et R'₁₁ qui représente (C₁-C₈)alkylène, éventuellement substitué par un ou des (C₁-C₈)alkoxy, l'autre n'étant pas I';
- p est un nombre entier de 0 à 5;
- R₄, R₅, R₆, R₇ et R₈ représentent, indépendamment, H, alkyle, alkoxyalkyle, amidoalkyle substitué ou non par alkyle ou alkoxyalkyle ou CH(R₁₂)X, R₁₂ étant H, alkyle, alkoxyalkyle ou R₁,
   ou les groupes R₄ et R₇ sont liés, et pris ensemble représentent 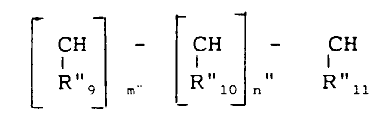 R''₉, R''₁₀, R''₁₁, m'' et n'' pouvant avoir les significations des lettres de même indice dans la formule I;
- B est O ou N-W et W représente comme R₅ ou polyoxy(C₂-C₃)alkylène, (C₁-C₆)alkylène-Y ou Y,
   Y étant un hétérocycle saturé ou non de 1 ou 2 cycles accolés, éventuellement substitué par un ou plusieurs OH, alkyle, alkoxy, alkoxyalkyle, ayant jusqu'à 12 sommets, comportant 1 à 4 hétéroatomes choisis parmi O, N, S, étant entendu que lorsque W représente Y, le carbone lié à N est lié à 2 atomes de carbone de l'hétérocycle, ou W représente la formule 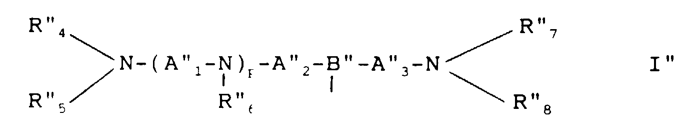 dans laquelle les lettres peuvent avoir les significations des lettres de même indice dans la formule I à l'exclusion de R''₉ et R''₁₁ qui ne peuvent représenter I' et de B'' qui représente N-Q, Q étant (C₁-C₈)alkylène, éventuellement substitué par un ou des alkoxy,
- ou A₂-B-A₃ représente un groupe hétérocyclique dans lequel B est un hétérocycle saturé ou non, de 5 ou 6 sommets comportant 1 ou 2 hétéroatomes choisis parmi O, S, N et A₂ et A₃ représentent un groupe CH-Rₑ dans lequel Rₑ est H ou (C₁-C₆)alkyle,
   étant entendu qu'au moins 3 groupes parmi R₄, R₅, R₆, R₇, R₈ et W représentent CH(R₁)X.

Notamment pour complexer les ions lanthanides, on préfère les composés de formule I qui comportent 3 atomes d'azote différents substitués par au moins un groupe CH(R₁)X, et mieux par des CH(R₁)X identiques avec X = CO₂H.

Un premier ensemble de ligands préférés parmi ceux de formule I est constitué des macrocycles de formule 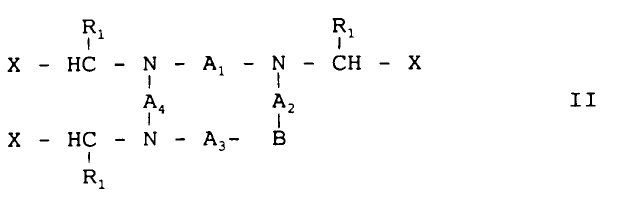 dans laquelle
- les groupes R₁ sont de préférence identiques, et les X représentent de préférence CO₂H,
- A₁ à A₄ représentent indépendamment 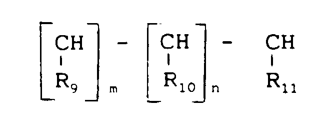 m et n étant des entiers de 0 à 2 dont la somme vaut 1 ou 2 et R₉, R₁₀ et R₁₁ représentent indépendamment H, alkyle, alkoxyalkyle, phényle, alkylènephényle, R₁₀ pouvant représenter aussi OH ou alkoxy ou l'un des R₉ et R₁₁ représente la formule 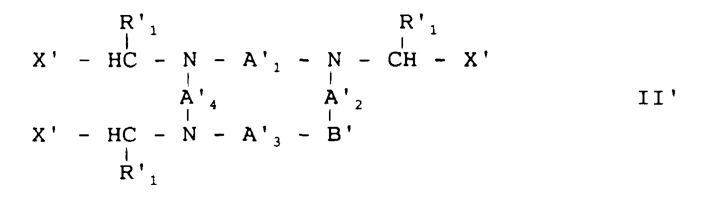 dans laquelle les lettres peuvent avoir les significations des lettres de même indice de la formule II, à l'exclusion de R'₉ ou R'₁₁ qui est lié au macrocycle II et représente (C₁-C₈)alkylène, éventuellement substitué par alkoxy,
- B représente N-W et W représente comme R₅ ou H, alkyle, alkoxyalkyle, amidoalkylène éventuellement substitué, polyoxy (C₂-C₃)alkylène, ces groupes comportant éventuellement un phényle, (C₁-C₆)alkylène-Y ou Y, Y étant un hétérocycle saturé ou non, de 1 ou 2 cycles accolés, éventuellement substitué par un ou plusieurs OH, alkyle, alkoxy, alkoxyalkyle, ayant jusqu'à 12 sommets, comportant 1 à 4 hétéroatomes choisis parmi O, N, S étant entendu que lorsque W représente Y,le carbone lié à N est lié à 2 atomes de carbone de l'hétérocycle,
   ou W représente la formule II' lorsque R₉ et R₁₁ en différent, dans laquelle les lettres peuvent voir les significations des lettres de même indice de la formule II à l'exclusion de B' qui représente N-(C₁-C₈)alkylène, éventuellement substitué par alkoxy, ou encore W représente CH(R₁)X,
   ou A₂-B-A₃ représente un groupe hétérocyclique dans lequel B est un hétérocycle saturé ou non, de 5 ou 6 sommets comportant 1 ou 2 hétéroatomes choisis parmi O, S, N et A₂ et A₃ représentent un groupe CH-Rₑ dans lequel Rₑ est H ou (C₁-C₆)alkyle.

On préfère les macrocycles dans lesquels A₁ à A₄ représentent (CH₂)₂ ou (CH₂)₃ ou l'un d'eux est substitué par R₁₁, R₁₁ représentant alkyle, phényle ou alkylènephényle, de préférence benzyle, éventuellement substitué et mieux ceux dans lesquels B est N-W.

A titre d'exemple de tels macrocycles, on peut citer 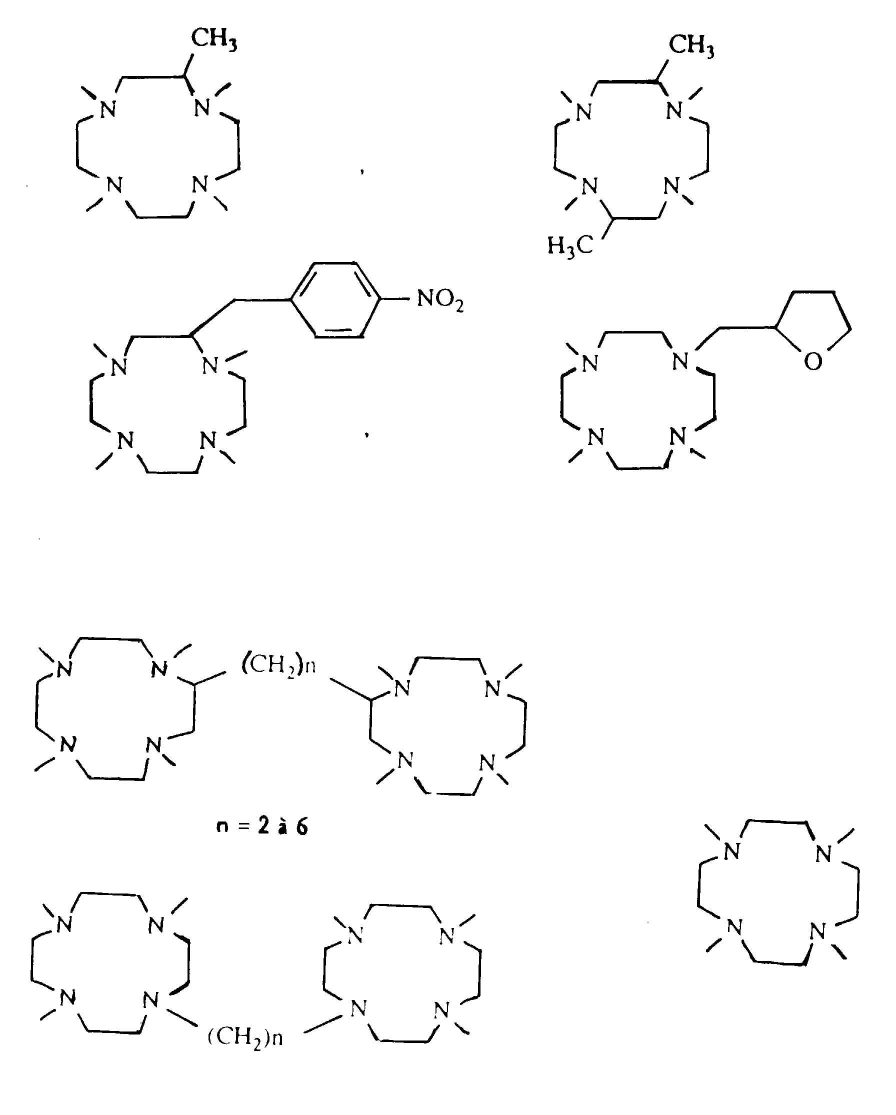 qui sont notamment décrits dans les références précédemment citées.

Dans le cas où un atome de carbone du macrocycle est substitué, on préfère notamment pour ne pas obtenir de mélange d'isomères, que les 4 atomes d'azote soient substitués par le même groupe CH(R₁)COOH.

Parmi les dérivés du 1, 4, 7, 10-tétraaza-cyclododécane, on préfère ceux de formule 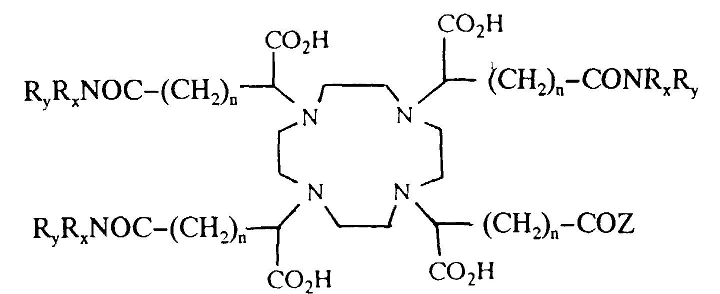 dans laquelle n est 2 ou 3 et
Rₓ est H, (C₁-C₁₄)alkyle éventuellement hydroxylé, et R_{y} est (C₂-C₁₄)alkyle hydroxylé, polyoxy (C₂-C₃)alkylène, polyhydroxyalkyle ou le reste d'un saccharide, d'un oligosaccharide ou d'un polysaccharide éventuellement monofonctionnalisés; R_{Y} peut comprendre aussi éventuellement des groupes (C₁-C₆)alkylène ou phénylène liés aux précédents par des fonctions amides ou éther-oxydes, et Z représente NRₓR_{Y} ou OH.

Parmi les dérivés de formule II, on peut citer aussi ceux de formule 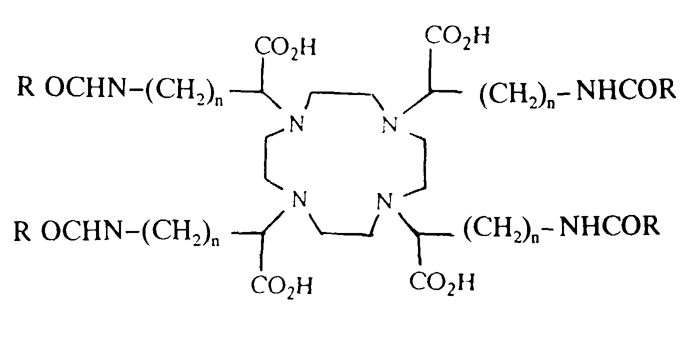 dans laquelle n est 2 ou 3
et R représente (C₂-C₁₄)alkyle hydroxylé;
polyoxy (C₂-C₃)alkylène ou un reste de saccharide, d'oligosaccharide ou de polysaccharide, éventuellement monofonctionnalisés.

Un autre ensemble de ligands préférés est celui des dérivés linéaires de formule 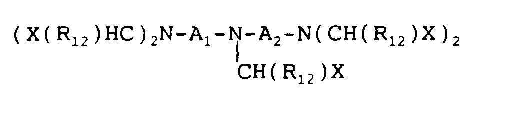 dans laquelle
- A₁ et A₂ représentent indépendamment 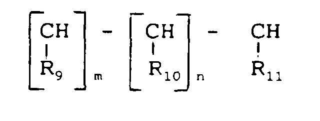 m et n étant 0, 1 ou 2 et leur somme valant 1 ou 2, R₉, R₁₀, R₁₁ représentant indépendamment H, alkyle, alkoxyalkyle, phényle, alkylènephényle, R₁₀ pouvant représenter aussi OH ou alkoxy ou l'un des R₉ et R₁₁ représente la formule 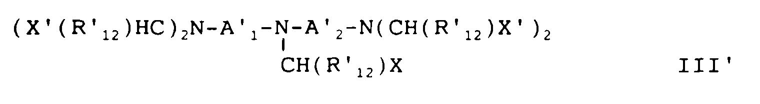 dans laquelle les lettres peuvent avoir les significations des lettres de même indice de la formule III à l'exclusion de R'₉ et R'₁₁ qui ne peuvent représenter III' et dont l'un représente (C₁-C₈)alkylène portant éventuellement un ou des alkoxy,
- R₁₂ représente H, alkyle, alkoxyalkyle ou R₁, étant entendu qu'au moins 3CH(R₁₂)X représentent CH(R₁)X, et de préférence sont identiques avec X étant CO₂H.

Selon un second aspect, l'invention concerne les complexes paramagnétiques formés entre les ligands de l'invention et les ions métalliques paramagnétiques convenables, tels que ceux du gadolinium, du dysprosium du manganèse, ainsi que les compositions d'agents de contraste pour l'imagerie médicale par résonance magnétique nucléaire qui comprennent ces complexes, associés aux véhicules et additifs usuels.

Les ligands selon l'invention peuvent aussi former des complexes avec des radioéléments comme ^{99m}Tc ou ⁹⁰Y, qui peuvent être utilisés pour établir un diagnostic ou effectuer un traitement thérapeutique.

Ces complexes se présentent, en général, sous forme de sel interne, résultat de la neutralisation par le cation métallique central de groupes acides du ligand; lorsque le complexe comprend d'autres groupes acides ceux-ci peuvent être salifiés par une base minérale ou organique pharmaceutiquement acceptable y compris les aminoacides, par exemple NaOH, lysine, N-méthylglucamine, arginine, ornithine ou diéthanolamine.

Les doses auxquelles les agents de contraste selon l'invention peuvent être administrés dépendent de la nature du complexe, de la relaxivité qu'il induit, de la voie d'administration et de l'organe visé. Par exemple par voie orale, notamment pour la sphère gastro-intestinale, on pourra administrer de 0,1 à 2 mM/kg et par voie parentérale de 0,001 à 1 mM/kg.

Selon un autre aspect, l'invention concerne un procédé de préparation des dérivés de poly(aminoacides) chélateurs qui consiste à faire réagir sur la polyamine qui en constitue le squelette, un réactif nucléophile de formule

Z'CH(R₁)-X

dans laquelle Z' représente un halogène ou un sulfonate et les groupes réactifs de R₁ et X sont éventuellement protégés, pour obtenir les atomes d'azote substitués conformément à l'invention, éventuellement après déprotection des groupes réactifs tels que les hydroxyles et les acides.

Comme dans les substitutions nucléophiles classiques, la réaction peut être effectuée dans un solvant aprotique polaire ou non tel qu'acétonitrile, diméthylformamide, toluène ou dans l'eau ou un alcool pur ou aqueux en présence d'une base minérale, tel qu'un hydroxyde ou carbonate alcalin ou alcalino-terreux, ou d'une amine tertiaire, à une température comprise entre la température ambiante et celle de reflux du solvant.

Lorsque tous les atomes d'azote de la polyamine ne doivent pas porter des substituants CH(R₁)X identiques, on peut effectuer des N-alkylations sélectives successives.

Par exemple, dans le cas du 1,4,7,10-tétraazacyclododécane, on peut effectuer une monoalkylation en faisant réagir un net excès du macrocycle sur Z'(CH(R₁)X dans des conditions opératoires, convenablement choisies comme décrit dans J. Org. Chem. 58, 3869-3876 (1993) ou en bloquant 3 des N par action de l'orthocarbonate d'éthyle ou d'un acétal de diméthylformamide, comme décrit dans J. Chem. Soc. Chem. Com. 1317-18 (1991); lorsque l'on effectue l'hydrolyse du composé obtenu sans avoir substitué le N non bloqué, on obtient un monoformamide et peut effectuer la trialkylation des 3 autres N.

On peut aussi obtenir des composés dissymétriques par un choix convenable des réactifs conduisant à la préparation du squelette polyamine; des exemples de ces réactions sont donnés dans EP-299 795, pour la préparation de dérivés linéaires ou cycliques.

Dans le cas de certains substituants R₁, notamment ceux de formule R₂-G-R₃, dans lesquels R₃ est une macromolécule et G est un groupe amido, il est avantageux de préparer les dérivés selon l'invention par l'intermédiaire de composés de formule I dont les atomes d'azote comportent des substituants de formule CH(R'₁)X, R'₁ étant de faible masse moléculaire, comportant de 2 à 5 carbones environ.

Ces intermédiaires de synthèse sont un autre objet de l'invention.

Ces dérivés sont représentés par les formules I, II, III, pour lesquelles les significations des lettres sont identiques à celles mentionnées précédemment à l'exclusion de celle de CH(R₁)X qui est CH(R₂-G')X', G' étant un groupe fonctionnel réactif, précurseur de G dont COOR', SO₃R', PO₃R', NHR' , SO₂NHR', N=C=S, N=C=O, OH et X' représente X ou X protégé, notamment sous forme d'ester.

Par groupe précurseur de G, on entend tous les groupes fonctionnels connus pour permettre la formation d'une liaison covalente dans des conditions opératoires accessibles dans l'industrie et par exemple, outre les groupes précédents, ceux utilisés pour les greffages sur des protéines.

Ces dérivés peuvent être préparés, comme précédemment décrit pour les dérivés de l'invention de formule I, mais avec les réactifs nucléophiles Z'CH(R₂G')X'.

Le couplage de ces intermédiaires aux dérivés réactifs de R₃ pour donner le ligand selon l'invention, dont au moins 3N portent un substituant CH(R₂-G-R₃)X, peut être réalisé selon des méthodes classiques, notamment celles couramment employées dans les synthèses peptidiques, ou encore après activation des acides sous forme d'halogénures d'acide, d'anhydrides ou en présence d'un agent déshydratant tel que les carbodiimides; selon la nature de G' et du groupe réactif de R'₃, on pourra effectuer une alkylation ou une acylation d'amine, ou la condensation d'un aldéhyde sur une amine suivie d'une réduction.

Parmi ces intermédiaires de synthèse, on peut citer ceux de formule 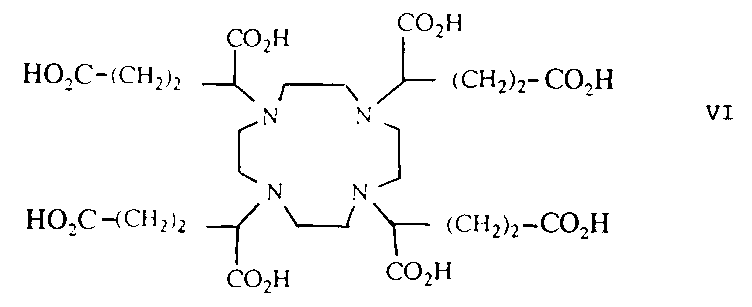 et les dérivés de formule 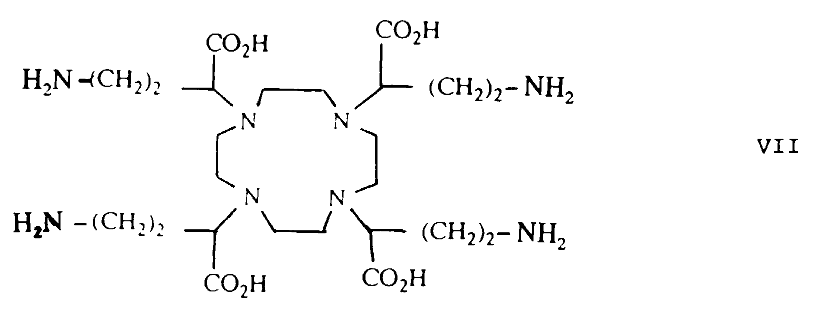 et leurs esters d'alkyle ou sels.

Les composés de formule VI sont particulièrement intéressants, en ce qu'ils permettent d'obtenir des tri- ou tétraamides dérivés des carboxyles sur le C en γ de l'azote sans modification des CO₂H en α, lorsque la réaction d'amidification est effectuée par action d'un chélate de VI sur une amine en présence d'un agent déshydratant tel qu'un carbodiimide en milieu aqueux ou organique.

Selon les conditions opératoires, proportions relatives de réactifs, solvant, durée et température de la réaction, et la réactivité de l'amine mise en oeuvre, on obtient un composé de formule VIII ou IX, ou leurs mélanges. 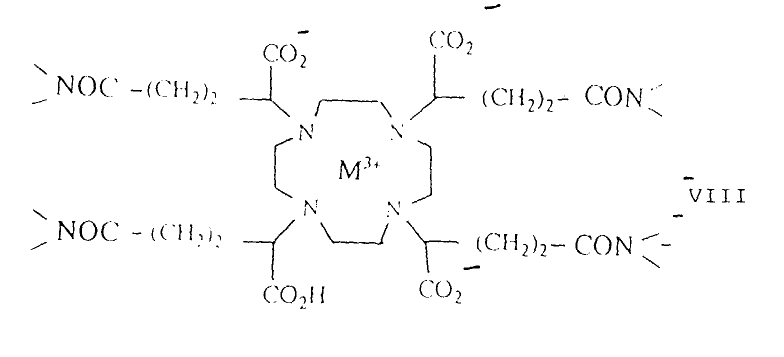 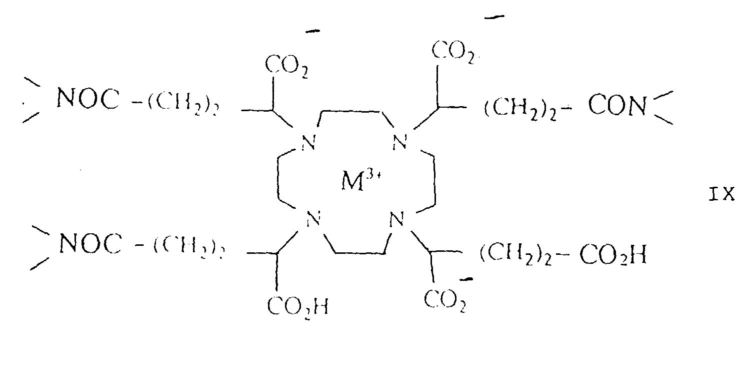 dans lesquels M³⁺ est de préférence Gd³⁺, ce qui permet d'obtenir directement le complexe utile comme agent de contraste, mais M³⁺ pourrait être tout cation chélaté par le ligand de formule VI; M³⁺ devrait alors, être séparé des ligands VIII ou IX par action d'un acide tel que HCl, H₂S ou HCN, les ligands étant ensuite mis à réagir avec un oxyde ou un sel de l'élément paramagnétique à complexer.

Il est évident que ce procédé peut être appliqué pour la préparation d'amides à d'autres composés dont le bras latéral comporte une fonction acide et X est CO₂H ou PO₃H.

En effet, il permet la protection sélective des différents acides et amines impliqués dans la coordination du métal.

Les complexes métalliques des dérivés de formule I, et ceux des intermédiaires de synthèse de formule VI peuvent être préparés de façon classique par action d'un équivalent de l'oxyde ou d'un sel du métal en milieu aqueux de préférence à une température supérieure à 20°C mais inférieure à 90°C.

Dans ce qui suit, on décrit des exemples de préparation de composés intermédiaires et de ligands ou chélates selon l'invention.

### EXEMPLES

### EXEMPLE 1

**Préparation du composé de formule**

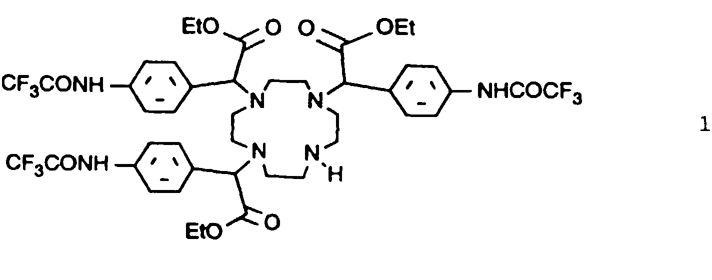

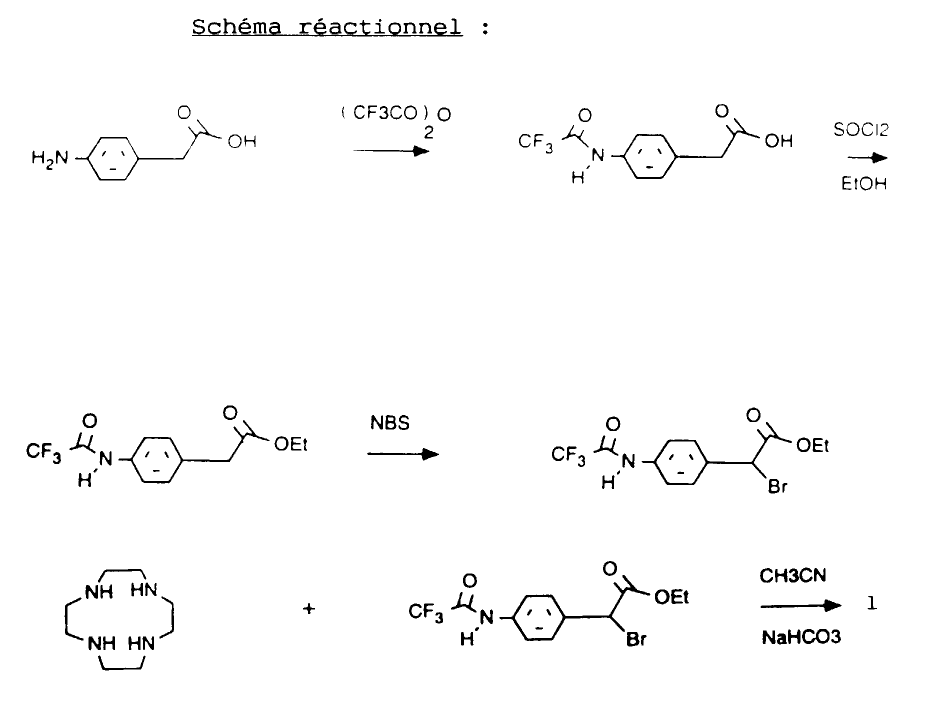

### 1. Acide 4-(trifluoroacétamido)benzèneacétique

Ce composé est préparé selon la méthode décrite par K.D. JANDA et coll. (J. Am. Chem. Soc. 113 n° 1, p. 291 (1991)) avec un rendement de 75%.

10 g d'acide 4-aminophénylacétique donnent 12 g de dérivé trifluoroacétylé caractérisé par RMN¹H. δ ppm (DMSO) : 7,45 (d, 4H) ; 7,56 (s, 2H) ; 11,15 (s, 1H).

### 2. 4-(trifluoroacétamido)benzèneacétate d'éthyle

Ce composé est obtenu au départ de l'acide précédemment préparé selon la méthode décrite par K.D. JANDA (J.A.C.S. 113 n° 1, p. 291 (1991)) avec un rendement de 33%. 12 g d'acide sont convertis en 4,4 g d'ester qui sont caractérisés en RMN¹H (δ ppm) DMSO : 1,1 (t, 3H) ; 3,6 (s, 2H) ; 4,05 (q, 2H) ; 7,4 (q,4 H).

### 3. α-Bromo 4-(trifluoroacétamido)benzèneacétate d'éthyle

4 g (14,5 mmol) d'ester précédemment préparés sont mis en suspension dans CCl₄ (150 cm³). Le mélange est agité et porté à léger reflux. 2,8 g de N-bromo succinimide et 0,2 cm³ de solution concentrée d'acide bromhydrique (38%) sont introduits dans le réacteur et le milieu est agité, à reflux, durant 48 h. L'insoluble est filtré et le solvant est évaporé. Le résidu est purifié sur silice (éluant CH₂ Cl₂) pour conduire à 2g de produit purifié.
Rendement : 40%
RMN¹H (δ ppm) :1,2 (t, 3H) ; 4,1 (q, 2H) ; 5,9 (s, 1H) ; 7,6 - 7,8 (m, 4H) ; 11,4 (s, 1H).

### 4. Préparation du composé 1

A une solution de 60 mg (0,35 mmol) de 1,4,7,10-tétraazacyclododécane dans 10 cm³ d'acétonitrile sont ajoutés 120 mg de NaHCO₃ et 500 mg de dérivé bromé précédemment préparé. La suspension est agitée à une température de 40°C durant 48 h. Le milieu réactionnel est ensuite filtré et le solvant évaporé sous pression réduite. Le résidu est repris à l'éther isopropylique pour donner 600 mg de produit brut sous forme de poudre. Le produit est purifié par chromatographie sur colonne de silice (éluant : AcOEt/MeOH 90/10 puis 80/20).
Masse obtenue : 110 mg
Rendement : 32%
RMN ¹³C (δ ppm) CDCl₃ : 14,6 ; 61,7 ; 63-67 ; 116 (CF₃) ; 130,6-136 (C aromatiques) ; 155 (CONH) ; 172 CO.

Les fonctions amines du composé 1 sont ensuite déprotégées par action de NaBH₄ dans l'éthanol comme décrit dans Chem. Ber. 103, 2437 (1970).

On obtient ainsi un composé intermédiaire de l'invention, pour lequel R₂ = C₆H₄ et G' est NH₂, précurseur d'un composé qui comporte 3 substituants, selon l'invention.

### EXEMPLE 2

**Préparation du composé de formule**

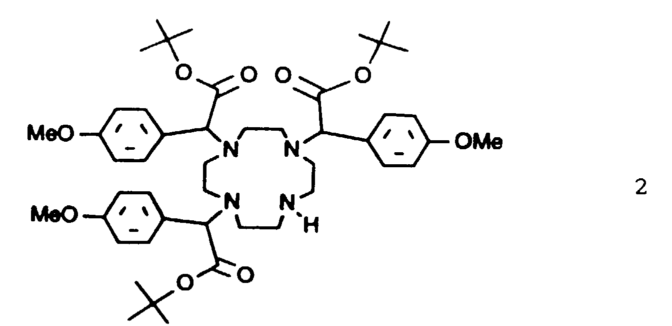

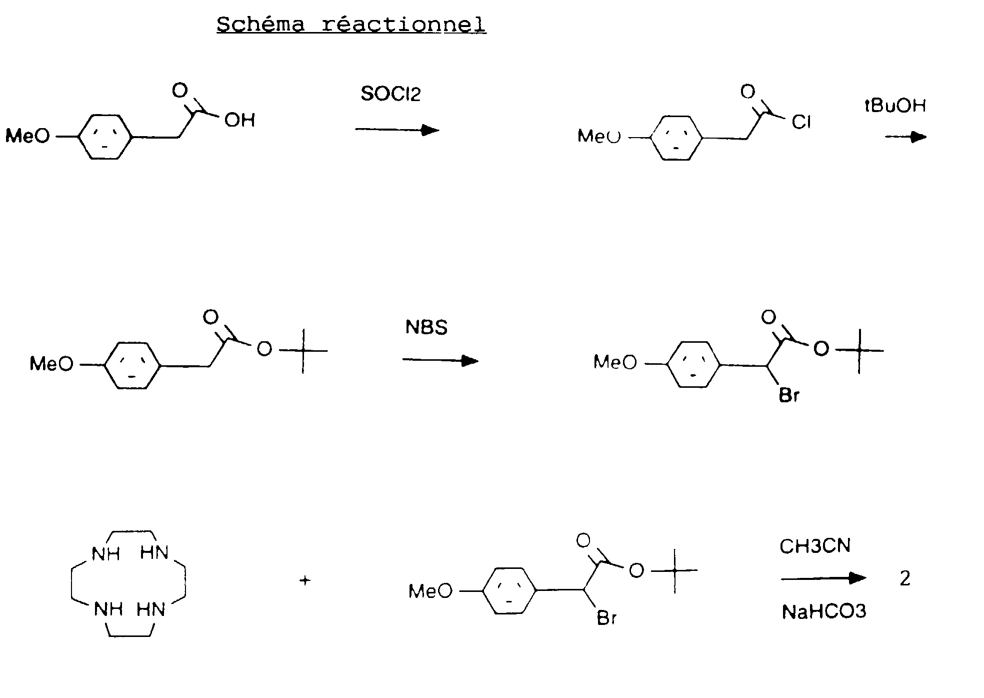

### 1. paraméthoxyphénylacétate de t-butyle

Le produit est préparé selon la méthode décrite par H. GOTTHARDT et coll. (Chem. Ber. 109, p. 740 (1976)) et P.G. NATTINGLY (J. Org. Chem. 46 p. 1557 (1981)). Au départ de 15 g d'acide paraméthoxyphénylacétique, 5 g d'ester t-butylique sont obtenus.
Rendement : 28%
RMN¹H (δ ppm) : 1,3 (9H, s) ; 3,46 (2H, s) ; 3,75 (3H, s) ; 6,8 - 7,3 (5H, q).

### 2. α-bromoparaméthoxyphénylacétate de t-butyle

Le produit est préparé selon la méthode décrite par H. GOTTHARDT et coll. et P.G. MATTINGLY.

5 g d'ester t-butylique précédemment préparés conduisent à 2,5 g de dérivé bromé.
Rendement : 33%
RMN¹H (δ ppm) : 1,48 (9H, s) ; 3,82 (3H, s) ; 5,28 (1H, s) ; 6,83 - 7,6 (5H, m).

### 3. Préparation du composé 2

600 mg de NaHCO₃ sont ajoutés à une solution constituée de 285 mg de 1,4,7,10-tétraazacyclododécane (1,65 mmol) dans 30 cm³ d'acétonitrile sous agitation. 2 g d'ester α-bromé précédemment préparés sont introduits à la suspension et le milieu réactionnel est agité 48 h à température ambiante. Après filtration et évaporation du solvant, le résidu est purifié par chromatographie sur silice (éluant : CH₂Cl₂ / AcOEt/MeOH 80/10/4) pour conduire à 500 mg de produit purifié.
Spectre de masse (FAB⁺) : pic 833
RMN¹³C (δ ppm) 27,6 (CH₃ t-butyle) ; 45-50 (cycle) ; 54,9 (C₃O); 61,1 (t-butyle); 68 (NCH) ; 113,6 - 127,2 - 130,4 - 158 (C aromatiques) ; 171 (C = O).

Les fonctions phénols du composé 2 sont ensuite libérées par action du tribromure de bore comme décrit dans Org. Synth. coll. vol. V, 412 (1973) ou dans J. Org. chem. 44, 4444 (1979).

On obtient ainsi un composé intermédiaire de l'invention pour lequel R₂ = C₆H₄ et G' est OH, qui peut être substitué de façon classique.

### EXEMPLE 3

**Préparation du composé de formule** 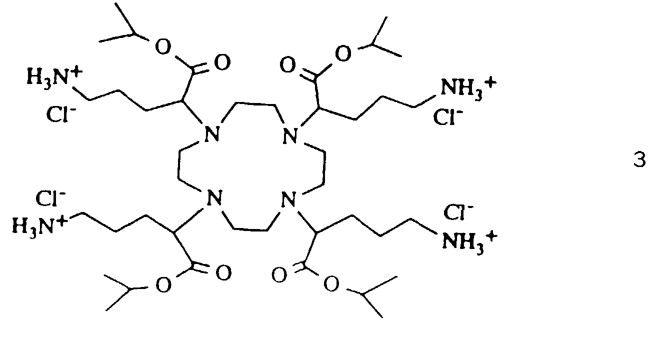 de l'acide correspondant et du complexe avec Gd³⁺

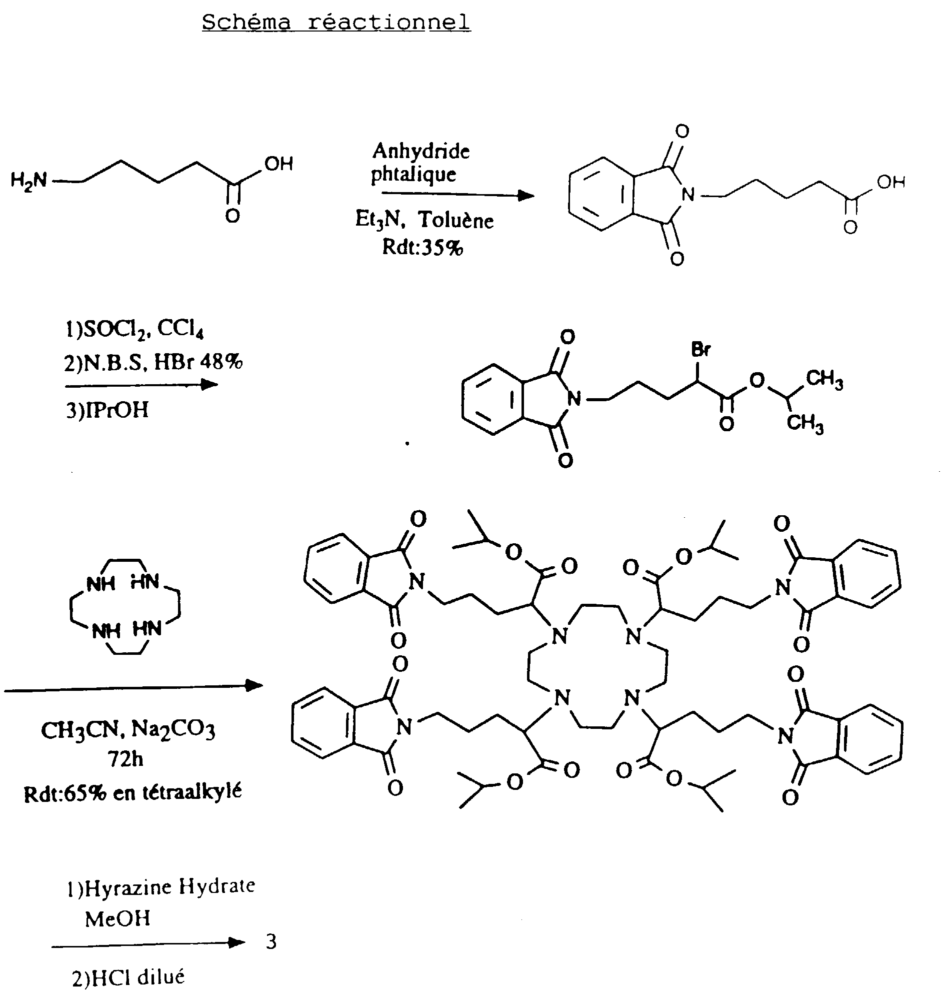

### 1. Acide 5-(N-phtalimido)-pentanoïque

12,6 g (85,1 mmole) d'anhydride phtalique, 10 g (85,1 mmole) d'acide 5-amino-valérique, 1,2 ml (8,51 mmole) de triéthylamine et 130 ml de toluène sont mélangés et agités à reflux 1 h dans un tricol équipé d'un Deanstark pour éliminer l'eau formée par azéotropie. Après une nuit à température ambiante, le précipité formé est filtré, lavé avec de l'heptane, puis 200 ml d'une solution d'acide chlorhydrique 1N, puis 100 ml d'eau. Après séchage, 7,37 g d'acide 5-(N-phtalimido)-pentanoïque sont obtenus sous forme de cristaux blancs avec un rendement de 35% (MP = 115°C).
RMN¹H (CDCl₃) δ(ppm) : 7,8 (m, 2H) ; 7,7 (m, 2H) ; 3,7
(t, 2H) ;2,4 (t, 2H) ; 1,7 (m, 4H) ;
¹³C (CDCl₃) : δ(ppm) :178 ; 168 ; 134 ; 133,9 ; 123,2 ; 37,4 ; 33,3 ; 27,9 ; 21,8.

### 2. Ester isopropylique de l'acide 2-bromo-5-(N-phtalimido)-pentanoïque

7,2 g (29,1 mmole) d'acide 5-(N-phtalimido)-pentanoïque sont ajoutés à une solution de 3 ml de tétrachlorure de carbone et 8,5 g (116 mmole) de chlorure de thionyle. La solution est portée à reflux 1 heure ; 14 ml de tétrachlorure de carbone, 6,2 g (34,9 mmole) de N-bromo succinimide et 2 gouttes d'acide bromhydrique aqueux à 48% sont ajoutés, et la solution est laissée à reflux 2 heures. La solution refroidie est versée sur 60 ml d'isopropanol et agitée 30 minutes. Après évaporation sous vide, l'huile obtenue est purifiée sur silice en éluant avec un mélange 50 dichlorométhane / 50 heptane puis dichlorométhane. Après évaporation des solvants, 8,2 g d'ester isopropylique de l'acide 2-bromo-5-(N-phtalimido)-pentanoique sont obtenus avec un rendement de 76,6% sous forme d'une huile jaune pâle qui cristallise (M.P. : 75°C).
RMN¹H (CDCl₃) : δ(ppm) : 7,85 (m, 2H) ; 7,7 (m, 2H), 5 (h, 1H) ; 4,2 (t, 1H) ; 3,7 (t, 2H) ; 1,7 - 2,2 (m, 4H) ; 1,2 (d, 3H) ; 1,25 (d, 3H).
¹³C (CDCl₃) : δ(ppm) :168,5 ; 168 ; 133,9 ; 132 ; 123,2 ; 69,7 ; 45,5 ; 36,9 ; 31,9 ; 26,9 ; 21,5 ; 21,3.

### 3. Tétraisopropylester de l'acide -1,4,7,10-tétraazacyclododécane-1,4,7,10-tétra-[2-(5-N-phtalimido) pentanoïque]

0,92 g (5,34 mmole) de 1,4,7,10-tétraazacy-clododécane, 11,8 g (32,1 mmole) du composé (2) et 3,4 g (32,1 mmole) de carbonate de sodium et 36 ml d'acétonitrile sont agités à reflux 72 h. Après filtration, évaporation sous vide, l'huile obtenue est reprise dans du dichlorométhane et lavée avec de l'eau. Après séchage et évaporation du dichlorométhane, le résidu obtenu est purifié par deux chromatographies flash sur silice, successives avec comme premier éluant un mélange 95 CH₂Cl₂/5 CH₃OH, puis comme deuxième éluant 95 CH₃COOC₂H₅5CH₃OH. Après évaporation des solvants, 4,62 g de tétraisopropylester de l'acide -1,4,7,10-tétraazacyclododécane-1,4,7,10-tétra-[2-(5-N-phtalimido) pentanoïque] sont obtenus avec un rendement de 65% sous forme de cristaux amorphes.
RMN¹H (CDCl₃) : δ(ppm) : 7,5 - 7,85 (m, 16H) ; 4,8 - 5,1 (m, 4H) ; 1 - 3,8 (m, 72 H)
¹³C (CDCl₃) : δ(ppm) : 167 ; 166,9 ; 162,8 ; 128,4 ; 126,9 ; 117,8 ; 62,2 ; 57,9 ; 45,3 ; 45 ; 32,5 ; 22,3 ; 20,4 ; 16,9 ; 16,7.

### 4. Ester tétraisopropylique de l'acide-1,4,7,10-tétraazacyclododécane-1,4,7,10-tétra-[2-(5-amino) pentanoïque], tétrachlorhydrate

1 g (0,76 mmole) de tétraisopropylester de l'acide-1,4,7,10-tétraazacyclododécane-1,4,7,10-tétra- [2-(5-N-phtalimido) pentanoïque], 0,15 ml d'hydrazine hydrate (3,04 mmole) et 8 ml de méthanol sont agités à reflux pendant 1 heure. A température ambiante, 10 ml d'acide chlorhydrique 0,5 M sont ajoutés. Le précipité formé est éliminé par filtration, et le filtrat est évaporé.

### 5. Acide 1,4,7,10-tétraazacyclododécane-1,4,7,10-tétra-[2-(5-amino)pentanoïque](chlorhydrate)

3,1 g du dérivé phtalimido obtenu selon 3 et 270 ml de solution aqueuse de HCl 6N sont maintenus au reflux, sous agitation pendant plusieurs jours; on concentre alors sous pression réduite jusqu'à 20ml, sépare le solide, extrait à l'éther éthylique et amène à sec. Le résidu est purifié par chromatographie sur silice silanisée, en éluant à l'eau; la solution aqueuse du produit recherché est concentrée et le résidu concrétisé dans l'éthanol, pour donner 1,15 g de l'acide. F =250°C
RMN¹H (D₂O) δppm : 3,8-4 (m,4H); 2,8-3,6 (m,24H); 1,5-2,2 (m,16H)

### 6. Complexe de l'acide précédent avec Gd³⁺

1,2 g du produit précédent et 0,5 g de GdCl₃, 6H₂O sont dissous dans 17 ml d'eau. Le pH du milieu évolue avec la réaction; il est maintenu à 6 par addition d'une solution aqueuse de NaOH 1N; lorsque le pH est stabilisé à 6, une nouvelle addition de NaOH, l'amène à 7 avant concentration sous pression réduite. Le solide obtenu est concrétisé dans l'éthanol à 75% (V/V).

On obtient ainsi 1,1 g du produit recherché sous forme de cristaux beiges qui fondent à plus de 300°C.

### EXEMPLE 4

1,4,7,10-tétrakis {3-[N(2-hydroxyéthyl)-N-(1-déoxyglucitol)-carboxamido]-1-carboxypropyl}-1,4,7,10-tétraazacyclododécane (complexe de gadolinium, sel de Na composé n° 4).

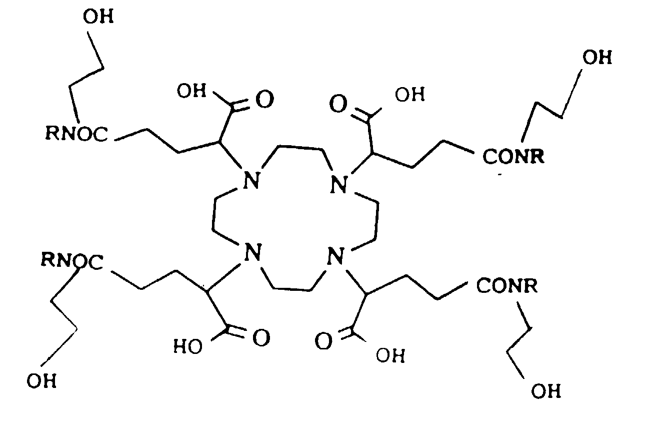

R = CH₂ - (CH₂OH)₄ - CH₂OH

### 1. 1,4,7,10-tétrakis-[N,N',N'',N'''-1-(1,3-diméthoxycarbonyl-propyl)]-1,1,4,7,10-tétraazacyclododécane

43 g (0,18 mol) de diméthyl 2-bromoglutarate préparés selon T.R. HOYE J. Org. Chem. 1982, 47, 4152-4156 sont ajoutés goutte à goutte à un mélange de 4,3 g (0,025 mol) de 1,4,7,10-tétraazacyclododécane, 25 g (0,18 mol) de carbonate de potassium, 100 ml d'acétonitrile, à 50°C. La suspension est agitée 48 heures à cette température, puis filtrée. Après évaporation à sec de l'acétonitrile, le résidu est purifié deux fois par chromatographie flash sur silice avec un gradient de dichlorométhane-méthanol. Après évaporation des solvants, 15 g de poudre beige sont obtenus avec un rendement de 75%.
CCM : SiO₂ CH₂Cl₂ - CH₃OH (9-1)
Rf = 0,8

### 2. 1,4,7,10-tétrakis-[N,N',N",N'''-1-(1,3-dicarboxypropyl)]-1,4,7,10-tétraazacyclododécane

15 g (0,019 mol) de composé 1,4,7,10-tétra-kis-[1,3 diméthoxycarbonyl-propyl]-1,4,7,10-tétraazacy-clododécane sont agités dans 100 ml de méthanol et 250 ml de solution aqueuse de NaOH N pendant 16 heures à température ambiante. L'octaacide en solution est purifié par fixation sur résine IRA 458 commercialisée par Rohm et Haas puis élution par un gradient d'acide acétique. Après évaporation des solvants, 11 g de poudre blanche sont obtenus avec un rendement de 85%.
CCM : SiO₂ CH₃COOC₂H₅-CH₃OH-CH₃COOH (35 - 35 -40)
Rf = 0,2
RMN¹³C DMSO (δ ppm) 31,13 ; 47,50 ; 61,13 ; 61,76 ; 172,29 ; 174,9 ;
Spectre de Masse (FAB') pic = 693

### 3. Complexe avec le gadolinium du composé intermédiaire précédent (pentasel de Na)

Une suspension de 12,1 g (0,0175 mol) du composé obtenu selon 2 et 6,5 g (0,0175 mol) de GdCl₃, 6H₂O dans 225 ml d'eau est amené à pH 6,5 par addition d'une solution aqueuse de NaOH 1N et maintenue à ce pH par des additions successives. Lorsque le pH n'évolue plus, l'eau est éliminée sous pression réduite pour donner 19,8g de poudre blanche, mélange du produit final avec NaCl.

### 4. Complexe de gadolinium du 1,4,7,10 tétrakis{3-[N-(2-hydroxyéthyl)-N-(1-déoxyglucitol)-carboxamido]-1-carboxypropyl}-1,4,7,10-tétraazacyclododécane, monosel de sodium (composé n° 4)

A une suspension de 2 g (2,9 mmol) du composé obtenu en 2 dans 100 ml d'eau avec 1,1 g (2,9 mmol) de chlorure de gadolinium (III) hexahydrate, à 80°C, est ajoutée une quantité suffisante de solution aqueuse de NaOH 0,1 N pour un pH de 4,3. La solution obtenue est amenée à pH 7 par ajout de la même solution de NaOH puis concentrée à un volume de 10 ml. Après ajout de 2,8 g (12,2 mmol) de 1-déoxy-1-(2-hydroxyéthylamino) D-glucitol, le pH est amené à 5,3 par addition d'acide chlorhydrique aqueux 1N et 2,3 g (12,2 mmol) de chlorhydrate de 1-(3-diméthylaminopropyl)-3-éthylcarbodiimide sont ajoutés, et la solution agitée 16 heures à température ambiante. La solution est amenée à pH 3,5 par addition de résine IRN 77 commercialisée par Rohm et Haas, filtrée, ajustée à pH 5,5 à l'aide de solution aqueuse de NaOH 0,1 N, puis éluée sur silice silanisée. Par évaporation de l'eau puis lavage du résidu deux fois par 100 ml d'éthanol et séchage, 4 g de poudre blanche sont obtenus.

### EXEMPLE 5

Complexe de gadolinium du composé n° 5 de formule 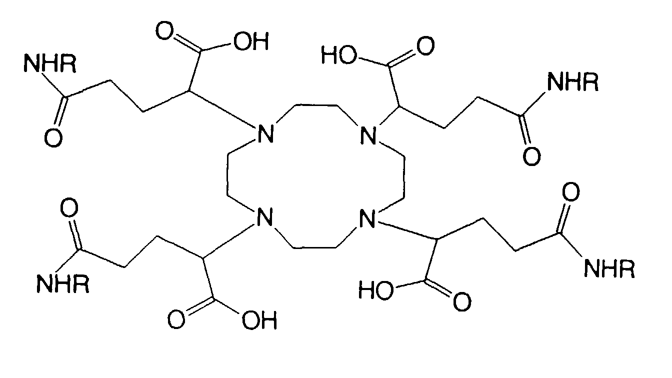 avec

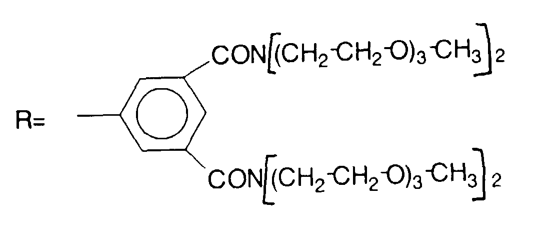

### 1. Préparation de R-NO₂

1g de chlorure de l'acide 5-nitroisophtalique sont introduits à 0°C dans une solution de 2,5 g de bis(trioxa-3,6,9)décylamine, préparée selon la méthode décrite dans Tetrahedron 47 411 (1991), et 1,12 ml de triéthylamine dans 10 ml de dichlorométhane. Le milieu est laissé 2 heures sous agitation à température ambiante puis lavé à l'eau, séché sur Na₂SO₂ et concentré. Le résidu obtenu est purifié par chromatographie sur silice en éluant avec un mélange de CH₂Cl₂/CH₃OH (95/5). On obtient ainsi
2,26 g du produit recherché, sous forme d'huile jaune.

### 2. 5-amino,N,N,N',N'-tétrakis(trioxa-3,6,9 décyl)1,3-phényldicarboxamide (RNH₂)

2,2 g de dérivé nitré précédent dans 10 ml d'éthanol sont hydrogénés, en présence de Pd/C à 10%, sous une pression de 10⁵ Pa à la température de 20°C. Après filtration et concentration sous pression réduite du milieu on obtient 2 g de l'amine sous forme d'huile jaune.
RMN¹H (CDCl₃) δ(ppm); 6,7 (s,2H); 6,6(s,1H); 3,35-3,7 (m,48H); 3,25 (s,12H).

### 3. Complexe du composé n° 5 avec Gd³⁺

1 g du complexe obtenu à l'étape 3 de l'exemple 4, 3,23 g de RNH₂ et 6,8 g de 1-(3-diméthylaminopropyl)-3-éthylcarbodiimide (chlorhydrate) sont dissous dans 13 ml d'eau; la solution est laissée 48 heures, sous agitation, à température ambiante avec une addition de temps en temps d'une solution aqueuse de HCl 1N pour maintenir un pH voisin de 7. Le milieu est amené à 150 ml par addition d'eau puis soumis à une ultrafiltration dans une cassette minisette de type nova, commercialisée par FILTRON (USA), avec une membrane de seuil de coupure 3 KDaltons.

Le produit recherché a un temps de rétention de 30 minutes, lors d'une gel-filtration dans une colonne Pharmacia de 60 cm x 2 cm remplie de gel Superdex^{R} 75, avec un débit d'éluant (H₂O) de 1 ml par minute.

### EXEMPLE 6

Complexe avec Gd³⁺ du composé n° 6 de formule (sel de sodium) 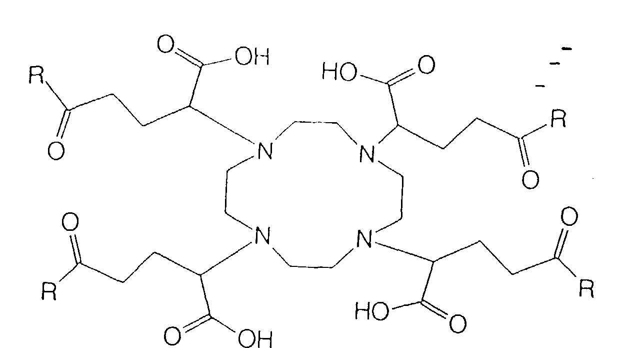 avec R = N(CH₂(CHOH)₄-CH₂OH)₂.

On dissout 1 mmol du complexe obtenu à l'étape 3 de l'exemple 4 et 4, 6 mmol de bis-(2,3,4,5,6-pentahydroxy hexyl)amine commerciale dans 13 ml d'eau; le pH de la solution est amené à 6 par addition d'une solution aqueuse de HC1 2N, puis on ajoute 21 mmol de chlorhydrate de 1-(3-diméthylaminopropyl)-3-éthyl-carbodiimide; après 4 heures d'agitation, on ajoute de nouveau 21 mmol du carbodiimide dans le milieu; après une nuit d'agitation on ajoute 100 ml d'eau et filtre la solution sur une résine IRN77, sous forme H⁺ commercialisée par Rohm et Haas, puis sur une résine IRA 458, sous forme OH⁻, commercialisée par Rohm et Haas; la solution finale est ultrafiltrée dans une cassette FILTRON, cassette FILTRON, équipée d'une membrane de 1 kDalton de seuil de coupure.

le produit final a un temps de rétention de 78 minutes dans une gel-filtration sur Superdex^{R} 30, avec un débit d'éluant (tampon phosphate pH = 7,2) de 1 ml/minute.

### EXEMPLE 7

Complexe avec Gd³⁺ du composé n° 7 de formule 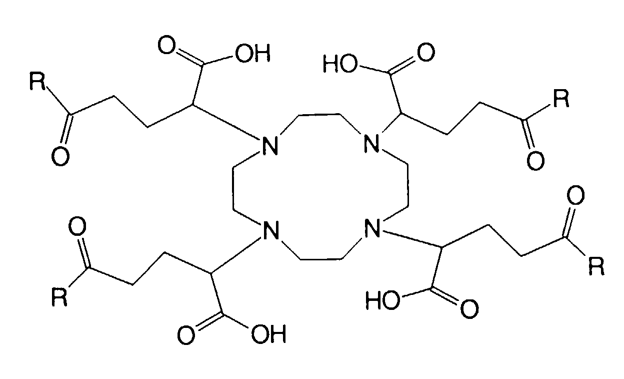 avec

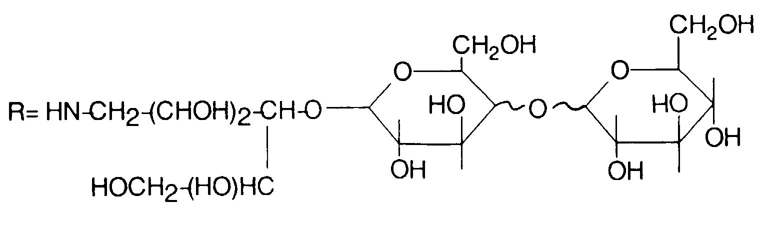

### 1. Préparation de RCH₂C₆H₅ puis RH d'après J. Carbohydrate Chemistry 11(7) 813-835 (1992)

On introduit 8,2 ml de benzylamine distillée dans une solution à 60°C de 23,6 g de maltotriose dans 16 ml d'eau. Après 3 heures d'agitation à cette température, on ajoute 60 ml de méthanol et amène le milieu vers 25°C avant d'ajouter par portions 3,56 g de borohydrure de sodium.

Après 48 heures d'agitation à 20°C, la solution est concentrée et le résidu dissous dans 100 ml de méthanol; on ajoute une solution aqueuse d'acide chlorhydrique 4N jusqu'à pH 3 et on concentre après addition de 2 volumes de méthanol. Le résidu est dissous dans 100 ml de méthanol, filtré, puis la solution est concentrée. Le solide résiduel est lavé à l'éthanol à 70°C puis séché pour donner 25,6 g de RCH₂C₆H₅,HCl. L'amine est libérée par action d'une résine IRA 458 commercialisée par Rohm et Haas et purifiée par passage sur une résine IRN77. On obtient ainsi 17,7 g de solide. CCM (silice Merck 60 F)
éluant : dioxane/eau/NH₃ aqueux 25% (p/V : 8/3/2)
Rf = 0,7

La benzylamine obtenue est dissoute dans 100 ml d'eau et on ajoute une solution aqueuse de NH₄OH à 25% jusqu'à pH 9. Après addition de 4 g de Pd/C le mélange est hydrogéné sous une pression de 6 x 10⁵Pa pendant 5 heures à 40°C et 12 heures à température ambiante.

Après filtration, on élimine le solvant sous pression réduite et on purifie l'huile par passage sur une résine IRN77, sous forme H⁺. On obtient 10,9 g du solide recherché.
CCM (conditions précédentes) Rf = 0,2
RNM¹³C (D₂O) : 40,6 (CH₂-NH₂); 57,7-59,5 (CH₂OH) 66,6-70,1 (CHOH); 74,1 et 79,2 (C-O); 97 et 07,6 (O-C-O).

### 2. Complexe du composé n° 7 avec Gd³⁺

On introduit à 60°C 4,66 g du produit précédemment obtenu dans 210 ml de diméthylformamide, puis lg du complexe de Gd³⁺ obtenu à l'exemple 4 (3), 886 mg d'hydroxy-1 benzotriazole, 1,25 g de chlorhydrate de 1-(3-diméthylaminopropyl)-3-éthylcarbodiimide et 0,9 ml de triéthylamine. Le milieu est maintenu 5 heures sous agitation à 60°C puis 48 heures à température ambiante, avant de concentrer sous pression réduite. Le résidu est trituré dans CH₂Cl₂ puis purifié par ultrafiltration sur une mini-cassette FILTRON avec une membrane de seuil de coupure 1 kDalton.

### EXEMPLE 8

Complexe avec Gd³⁺ du composé n° 8 de formule 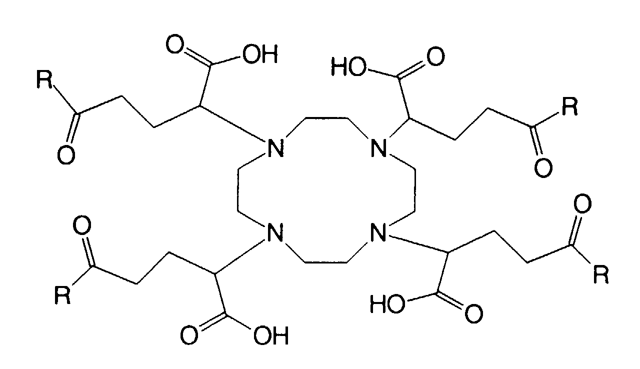 avec R = HN(CH₂CH₂O)ₙ-CH₃

L'éther méthylique de l'aminopolyéthylène glycol (MM ≃ 5000) peut être préparé selon l'une des méthodes précédemment citées ou acheté dans le commerce.

15 g de l'amine sont dissous à 40°C dans 700 ml de diméthylformamide et une solution de 0,5 g du complexe de Gd³⁺ préparé à l'exemple 4 (3) dans 50 ml d'eau sont ajoutés, puis 0,48 g d'hydrate d'hydroxybenzotriazole, 0,5 ml de triéthylamine et 2,72 g de chlorhydrate de 1-(3-diméthylaminopropyl)-3-éthylcarbodiimide.

Après 5 jours d'agitation à température ambiante, on concentre la solution sous pression réduite. Le résidu, dissous dans 150 ml d'eau, est ultrafiltré dans une minicassette FILTRON avec une membrane de seuil de coupure 5 kDaltons.

Après lyophilisation, on isole 3,5 g de produit, mélange de triamide (l'un des R = OH) et de tétraamide.

## Revendications

1. Dérivés de polyaminoacides, chélateurs d'ions métalliques paramagnétiques, de formule I 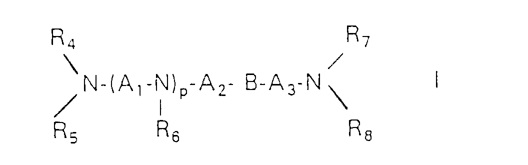 ou de formule II 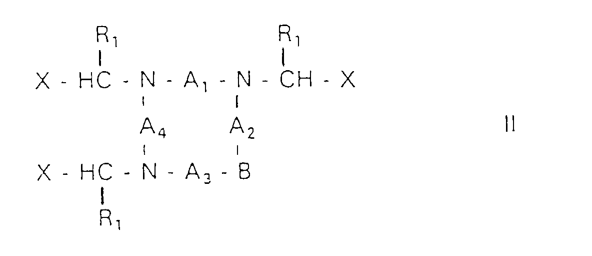 dans lesquelles
- A₁, A₂, A₃ et A₄, identiques ou différents, représentent 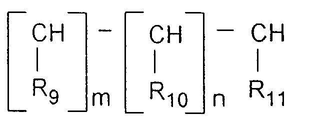
m et n étant des entiers de 0 à 5 dont la somme vaut 1 à 5 dans la formule I et des entiers de 0 à 2 dont la somme vaut 1 ou 2 dans la formule II,
R₉, R₁₀, R₁₁ représentent indépendamment H, alkyle, alkoxyalkyle, phényle, alkylènephényle, R₁₀ pouvant représenter aussi OH ou alkoxy ou l'un des R₉ et R₁₁ de la formule I représente 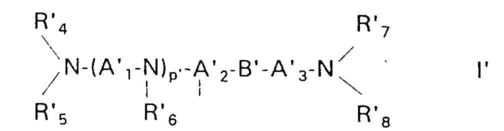 dans laquelle les lettres peuvent avoir les significations des lettres de même indice de la formule I, à l'exclusion de l'un des R'₉ et R'₁₁ qui représente (C₁-C₈)alkylène, éventuellement substitué par un ou des (C₁-C₈)alkoxy, l'autre n'étant pas l'
ou l'un des R₉ et R₁₁ de la formule II représente 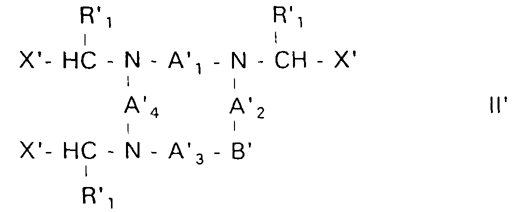 dans laquelle les lettres peuvent avoir les significations des lettres de même indice de la formule II, à l'exclusion de R'₉ ou R'₁₁ qui est lié au macrocycle Il et représente (C₁-C₈)alkylène, éventuellement substitué par un ou des alkoxy,
- R₄, R₅, R₆, R₇ et R₈ représentent, indépendamment, H, alkyle, alkoxyalkyle, amidoalkyle éventuellement substitué par alkyle ou alcoxyalkyle, ou CH(R₁₂)X, R₁₂ étant H, alkyle, alkoxyalkyle ou R₁ ou les groupes R₄ et R₇ sont liés et pris ensemble représentent 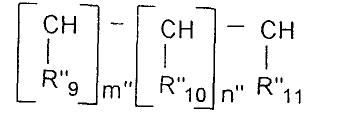 R"₉, R"₁₀, R"₁₁, m" et n" pouvant avoir les significations des lettres de même indice dans la formule I.
- B représente NCH(R₁)X ou N-W et W représente H, alkyle, alkoxyalkyle, amidoalkyle éventuellement substitué par alkyle ou alcoxyalkyle, ces groupes comportant éventuellement en outre un phényle dans le cas de la formule II, (C₁-C₆)alkylène-Y ou Y, Y étant un hétérocycle saturé ou non, de 1 ou 2 cycles accolés, éventuellement substitué par OH, alkyle, alkoxy, alkoxyalkyle, ayant jusqu'à 12 sommets, comportant 1 à 4 hétéroatomes choisis parmi O, N, S, étant entendu que lorsque W représente Y, le carbone lié à N est lié à 2 atomes de carbone de l'hétérocycle,
ou W représente le groupe I" dans la formule I : 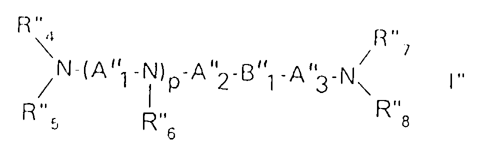 dans laquelle les lettres peuvent avoir les significations des lettres de même indice de la formule I à l'exception d'un des groupes R"₉ et R"₁₁ qui ne peut pas représenter I' et de B"₁ qui représente N-Q, Q étant (C₁-C₈)alkylène éventuellement substitué par un ou plusieurs alcoxy,
ou W représente le groupe II' dans la formule II dans lequel les lettres peuvent avoir les significations des lettres de même indice dans la formule II à l'exception de B'₂ qui représente N-(C₁-C₈)alkylène éventuellement substitué par un ou plusieurs alcoxy,
ou B est O dans la formule I
ou A₂-B-A₃ représente un groupe hétérocyclique dans lequel B est un hétérocycle saturé ou non, de 5 à 6 sommets comportant 1 ou 2 hetéroatomes choisis parmi O, S, N et A₂ et A₃ représentent un groupe CH-Rₑ dans lequel Rₑ est H ou (C₁-C₆)alkyle,
- p est un nombre entier de 0 à 5 ;
- les X, identiques ou différents, représentent CO₂Rₐ, CONR_{b}R_{c} ou P(R_{d})O₂H et Rₐ, R_{b}, R_{c}, identiques ou différents représentent H ou (C₁-C₈)alkyle, éventuellement hydroxylé ; R_{d} représente OH, (C₁-C₈)alkyle ou (C₁-C₈)alkoxy,
- et R1 représente un groupe hydrophile de masse moléculaire supérieure à 200, comportant au moins 3 atomes d'oxygène, de formule R₂-G-R₃ avec
R₂ représente rien, alkylène, alkoxyalkylène, polyalkoxyalkylène, alkylène interrompu par phénylène, phénylène ou un reste hétérocyclique saturé ou non;
G représente une fonction O, CO, OCO, COO, SO₃, OSO₂, CONR', NR'CO, NR'COO, OCONR', NR', NR'CS, CSNR', SO₂NR', NR'SO₂,NR'CSO, OCSNR', NR'CSNR', P(O)(OH)NR', NR'P(O)-(OH), dans laquelle R' est H, (C₁-C₈)alkyle ou R₃;
R₃ représente alkyle, phényle, alkyle substitué ou interrompu par un ou des groupes phényles, alkylèneoxy, amino ou amido substitués ou non par alkyle éventuellement substitué ou interrompu par l'un des groupes précédents ou R₃ est le reste d'un composé, éventuellement monofonctionnalisé, choisi parmi les saccharides et les oligosaccharides, les groupes phényles, phénylènes et hétérocycliques pouvant être substitués par OH, Cl, Br, I, (C₁-C₈)alkyle, (C₁-C₈)alkoxy, NO₂, NRₓR_{y}, NRₓCOR_{y}, CONRₓR_{y}, COORₓ, Rₓ et R_{y} étant H ou (C₁-C₈)alkyle et les groupes alkyle, alkylène, alkoxy, en C₁ à C₁₄, linéaires, ramifiés ou cycliques pouvant être hydroxylés, étant entendu qu'au moins 3 des atomes d'azote donneurs portent un groupe CH(R₁)X,
ainsi que les sels de ces dérivés avec des bases minérales ou organiques,

2. Dérivés selon la revendication 1, caractérisés en ce que X représente CO₂H, éventuellement salifié, et R₁ représente R₂-G-R₃ et R₂ représente (C₁-C₆)alkylène, éventuellement interrompu par phénylène, G représente CONR', NR'CO ou O, R' étant H, (C₁-C₈)alkyle ou R₃, et R₃ représente (C₁-C₁₄)alkyle éventuellement substitué ou interrompu par un ou des groupes phényle, (C₁-C₆)alkoxy, alkylèneoxy, amino et amido substitués ou non par alkyle éventuellement substitué ou interrompu comme précédemment, ou le reste d'un saccharide ou d'un oligosaccharide éventuellement monofonctionnalisé.

3. Dérivés selon l'une des revendications 1 ou 2, caractérisés en ce que les groupes CH(R₁)X sont identiques.

4. Dérivés selon la revendication 1 de formule II,
dans laquelle A₁, A₂, A₃, A₄ représentent (CH₂)ₙ avec n = 2, 3 ou l'un d'eux représente (CH₂)ₙ-CH-R₁₁ et les autres (CH₂)ₙ avec n' = n - 1 et R₁₁ représente alkyle, phényle ou alkylènephényle, éventuellement substitués, les groupes CH(R₁)X sont identiques et X est CO₂H et B représente N-W.

5. Dérivés selon la revendication 1 de formule 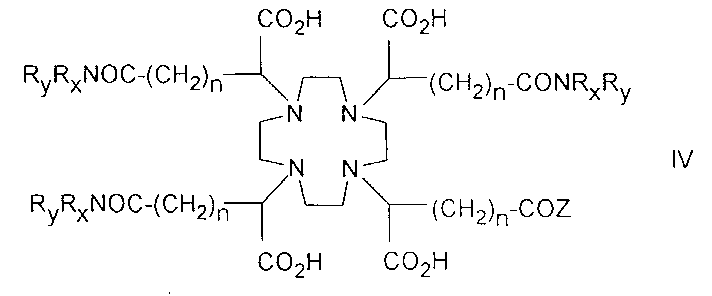 dans laquelle
Rₓ est H, (C₁-C₁₄)alkyle, éventuellement hydroxylé et R_{y} est (C₁-C₁₄)alkyle hydroxylé, ou le reste d'un monosaccharide ou d'un oligosaccharide, éventuellement monofonctionnalisé, R_{y} comportant éventuellement, en outre, des groupes (C₁-C₄)alkylène ou phénylène liés aux précédents par des fonctions amides ou étheroxydes,
n est 2 ou 3,
et Z représente NRₓR_{y} ou OH,
ainsi que leurs sels d'acide avec des bases minérales ou organiques.

6. Dérivés selon la revendication 1 de formule 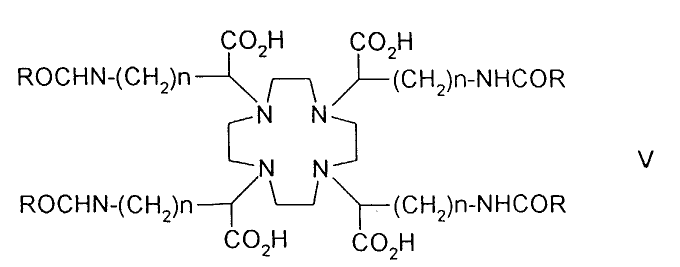 dans laquelle
n est 2 ou 3,
et R représente un reste de saccharide ou d'oligosaccharide, éventuellement monofonctionnalisé, et leurs sels d'acide ou de bases minéraux ou organiques.

7. Dérivés selon l'une des revendications 1 ou 2, de formule 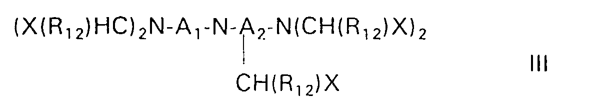 dans laquelle
- A₁, and A₂ représentent indépendamment 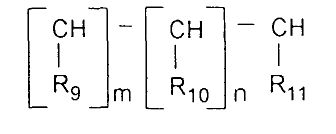
m et n étant 0, 1 ou 2 et leur somme valant 1 ou 2,
R₉, R₁₀, R₁₁ représentant indépendamment, H, alkyle, alkoxyalkyle, phényle, alkylènephényle, R₁₀ pouvant représenter en outre OH ou alkoxy ou l'un des R₉ et R₁₁ représente la formule 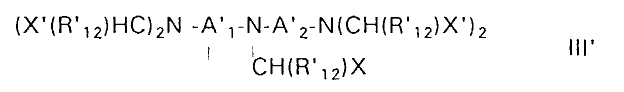 dans laquelle les lettres peuvent avoir les significations des lettres de même indice de la formule III à l'exclusion de R'₉ et R'₁₁ qui ne peuvent représenter III' et dont l'un représente (C₁-C₈)alkylène portant éventuellement un ou des alkoxy,
- R₁₂ représente H, alkyle, alkoxyalkyle ou R₁,
les phényles pouvant être substitués par un ou des groupes OH, Cl, Br, I, (C₁-C₈)alkyle, (C₁-C₈)alkoxy, NO₂, NRₓR_{y}, NRₓCOR_{y}, CONRₓR_{y}, COORₓ avec RₓR_{y} étant H ou (C₁-C₈)alkyle et les alkyle, alkylène, alkoxy étant linéaires ou ramifiés en C₁ à C₁₄ et éventuellement hydroxylés,
et leurs sels avec des acides ou des bases minérales ou organiques.

8. Dérivé de formule 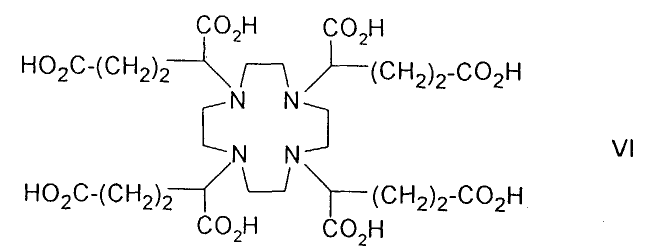 et ses chelates avec des cations métalliques et leurs sels avec des bases organiques ou minérales.

9. Dérivé de formule 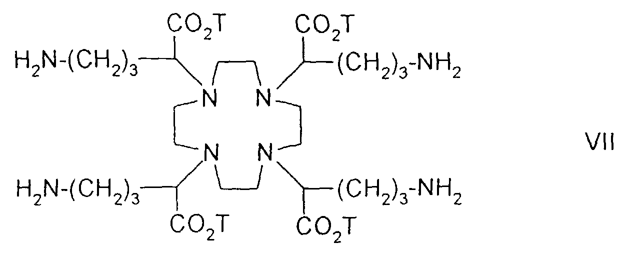 dans laquelle T représente H ou (C₁-C₈)alkyle et ses chelates avec des cations métalliques et leurs sels avec des acides organiques ou minéraux.

10. Chelate formé entre un ion métallique paramagnétique et un dérivé selon l'une des revendications 1 à 9.

11. Chelate selon la revendication 10, dans lequel l'ion est celui du gadolinium ou du manganèse.

12. Composition pour l'imagerie médicale par résonance magnétique nucléaire, caractérisée en ce qu'elle comprend un chelate selon l'une des revendications 10 ou 11 et un véhicule physiologiquement acceptable.

13. Procédé de préparation de chelates d'un dérivé de formule IV selon la revendication 5, qui consiste à faire réagir sur le chelate du dérivé de formule 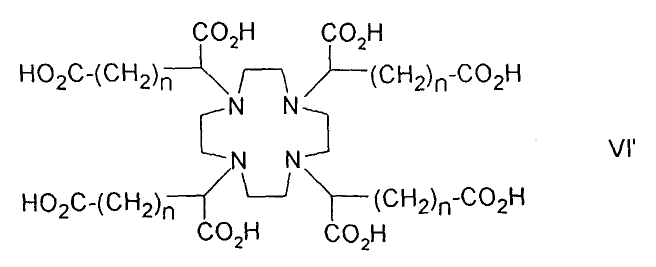 où n est 2 ou 3,
une amine de formule NRₓR_{y} dans laquelle Rₓ et R_{y} sont tels que définis à la revendication 5.

## Patentansprüche

1. Polyaminosäure-Derivate als Chelatbildner für paramagnetische Metallionen der Formel I 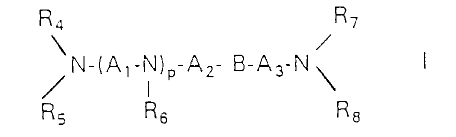 oder der Formel II 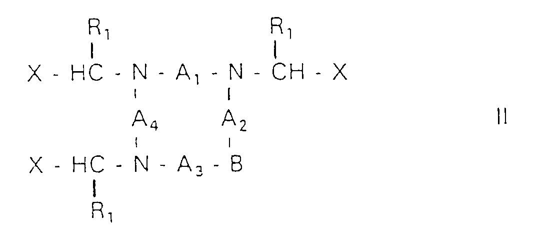 in denen
- A₁, A₂, A₃ und A₄, die gleichartig oder verschieden sind, 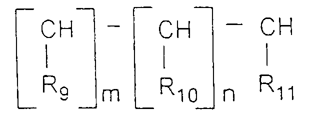 bedeuten, worin
m und n in der Formel I ganze Zahlen mit Werte von 0 bis 5 bedeuten, deren Summe 1 bis 5 beträgt, und in der Formel II ganze Zahlen von 0 bis 2 bedeuten, deren Summe 1 oder 2 beträgt,
R₉, R₁₀ und R₁₁ unabhängig voneinander H, Alkyl, Alkoxyalkyl, Phenyl, Alkylenphenyl bedeuten, worin R₁₀ auch OH oder Alkoxy bedeuten kann, oder einer von R₉ und R₁₁ der Formel I 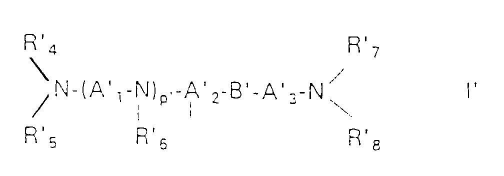 bedeutet, worin die Buchstaben die gleichen Bedeutungen besitzen wie die Buchstaben des gleichen Indexes der Formel I mit Ausnahme eines der Reste R'₉ und R'₁₁, der (C₁,C₈),Alkylen, gegebenenfalls substituiert durch ein oder mehrere (C₁-C₈)-Alkoxy, bedeutet, während der andere Rest nicht I' bedeutet,
oder einer der Reste R₉ und R₁₁ der Formel II 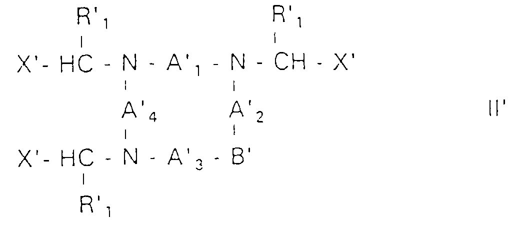 bedeutet,
in der die Buchstaben die Bedeutungen der Buchstaben des gleichen Indexes der Formel II besitzen können, mit Ausnahme von R'₉ oder R'₁₁, der an den Makrocyclus II gebunden ist und (C₁-C₈)-Alkylen, gegebenenfalls substituiert durch ein oder mehrere Alkoxy, darstellt,
- R₄, R₅, R₆, R₇ und R₈ unabhängig voneinander H, Alkyl, Alkoxyalkyl, Amidoalkyl gegebenenfalls substituiert durch Alkyl oder Alkoxyalkyl, oder CH(R₁₂)X, worin R₁₂ H, Alkyl, Alkoxyalkyl oder R₁ darstellt, bedeuten oder die Gruppen R₄ und R₇ verbunden sind und gemeinsam 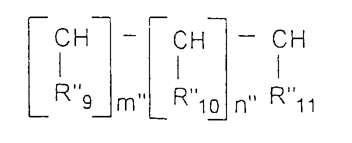 bedeuten,
worin R"₉, R"₁₀, R"₁₁, m" und n" die Bedeutungen der Buchstaben des gleichen Indexes der Formel I besitzen können,
- B NCH(R₁)X oder N-W darstellt, worin W H, Alkyl, Alkoxyalkyl, Amidoalkyl gegebenenfalls substituiert durch Alkyl oder Alkoxyalkyl, wobei diese Gruppen gegebenenfalls zusätzlich einen Phenylrest im Fall der Formel II tragen, (C₁-C₆)-Alkylen-Y oder Y bedeutet, wobei Y einen gegebenenfalls gesättigten Heterocyclus mit 1 oder 2 verbundenen Ringen darstellt, der gegebenenfalls durch OH, Alkyl, Alkoxy, Alkoxyalkyl substituiert ist, bis zu 12 Ringatome aufweist, 1 bis 4 Heteroatome ausgewählt aus O, N, S besitzt, wobei dann, wenn W Y darstellt, das an N gebundene Kohlenstoffatom an 2 Kohlenstoffatome des Heterocyclus gebunden ist,
oder W die Gruppe I" in der Formel I darstellt: 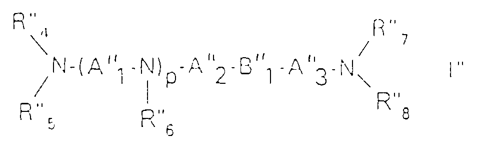
in der die Buchstaben die Bedeutungen der Buchstaben des gleichen Indexes der Formel I besitzen können mit Ausnahme einer der Gruppen R"₉ und R"₁₁, die nicht I' darstellen kann, und von B"₁, welches N-Q darstellt, worin Q (C₁-C₈)-Alkylen gegebenenfalls substituiert durch ein oder mehrere Alkoxy bedeutet, oder W die Gruppe II' in der Formel II darstellt, worin die Buchstaben die Bedeutungen der Buchstaben des gleichen Indexes der Formel II besitzen können mit Ausnahme von B'₂, welches N-(C₁-C₈)-Alkylen gegebenenfalls substituiert durch ein oder mehrere Alkoxy darstellt,
oder B in der Formel I O ist
oder A₂-B-A₃ eine heterocyclische Gruppe darstellt, worin B ein gegebenenfalls substituierter Heterocyclus mit 5 bis 6 Ringgliedern, die 1 oder 2 Heteroatome ausgewählt aus O, S und N aufweisen, darstellt und A₂ und A₃ eine Gruppe CH-Rₑ bedeuten, worin Rₑ H oder (C₁-C₆)-Alkyl darstellt,
- p eine ganze Zahl von 0 bis 5 bedeutet;
- die Reste X, die gleichartig oder verschieden sind, CO₂Rₐ, CONR_{b}R_{c} oder P(R_{d})O₂H bedeuten und Rₐ, R_{b} und R_{c}, die gleich oder verschieden sind, H oder gegebenenfalls hydroxyliertes (C₁-C₈),Alkyl darstellen; R_{d} OH, (C₁-C₈)-Alkyl oder (C₁,C₈)-Alkoxy arstellt,
- und R₁ eine hydrophile Gruppe mit einer Molekülmasse von mehr als 200 darstellt, die mindestens 3 Sauerstoffatome aufweist der Formel R₂-G-R₃, worin R₂ nichts, Alkylen, Alkoxyalkylen, Polyoxyalkylen, Alkylen unterbrochen durch Phenylen, Phenylen oder eine gesättigten oder ungesättigten heterocyclischen Rest darstellt;
G eine Funktion der Formeln O, CO, OCO, COO, SO₃, OSO₂, CONR', NR'CO, NR'COO, OCONR', NR', NR'CS, CSNR', SO₂NR', NR'SO₂, NR'CSO, OCSNR', NR'CSNR', P(O)(OH)NR', NR'P(O)-(OH) darstellt, worin R' H, (C₁-C₈)-Alkyl oder R₃ bedeutet;
R₃ Alkyl, Phenyl, Alkyl substituiert oder unterbrochen durch eine oder mehrere Phenylgruppen, Alkylenoxy, Amino oder Amido gegebenenfalls substituiert durch Alkyl, welches gegebenenfalls substituiert oder unterbrochen ist durch eine oder oben angegebenen Gruppen, darstellt oder R₃ der Rest einer gegebenenfalls monofunktionalisierten Verbindung ist, ausgewählt aus Sacchariden und Oligosacchariden,
wobei die Phenyl-, Phenylen und heterocyclischen Gruppen durch OH, Cl, Br, I, (C₁C₈)-Alkyl, (C₁-C₈)-Alkoxy, NO₂, NRₓR_{y}, NRₓCOR_{y}, CONRₓR_{y}, COORₓ substituiert sein können, worin Rₓund R_{y} H oder (C₁-C₈)-Alkyl bedeuten und die C₁-C₁₄-Alkylgruppen, C₁-C₁₄-Alkylengruppen und C₁-C₁₄-Alkoxygruppen, die geradkettig, verzweigt oder cyclisch sind, hydroxyliert sein können, mit der Maßgabe, daß mindestens 3 der Donor-Stickstoffatome eine Gruppe CH(R₁)X tragen,
sowie die Salze dieser Derivate mit anorganischen oder organischen Basen.

2. Derivate nach Anspruch 1, dadurch gekennzeichnet, daß X CO₂ H, gegebenenfalls in der Salzform, darstellt und R₁ R₂-G-R₃ darstellt und R₂ (C₁-C₆)-Alkylen, gegebenenfalls unterbrochen durch Phenylen, bedeutet, G CONR', NR'CO oder O bedeutet, worin R' H, (C₁-C₈)-Alkyl oder R₃ darstellt, und R₃ (C₁-C₁₄)-Alkyl gegebenenfalls substituiert oder unterbrochen durch eine oder mehrere Phenylgruppen, (C₁-C₆)-Alkoxy, Alkylenoxy, Amino und Amido gegebenenfalls substituiert durch Alkyl, welches gegebenenfalls substituiert oder unterbrochen ist wie oben angegeben, oder den Rest eines gegebenenfalls monofunktionalisierten Saccharids oder Oligosaccharids darstellt.

3. Derivate nach einem der Ansprüche 1 oder 2, dadurch gekennzeichnet, daß die Gruppen CH(R₁)X identisch sind.

4. Derivat nach Anspruch 1 der Formel II,
worin A₁, A₂, A₃ und A₄ (CH₂)ₙ, worin n = 2, oder 3 darstellt, oder eine dieser Gruppen (CH₂)ₙ-CH-R₁₁ und die anderen (CH₂)ₙ, bedeuten, worin n' = n - 1 und R₁₁ gegebenenfalls substituiertes Alkyl, Phenyl oder Alkylenphenyl darstellen, wobei die Gruppen CH(R₁)X identisch sind und X CO₂H und B N-W bedeuten.

5. Derivate nach Anspruch 1 der Formel 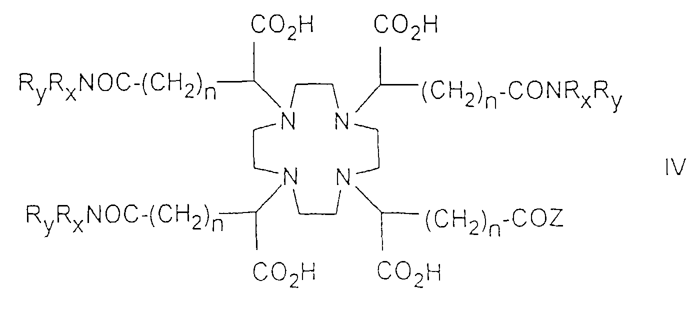 in der
Rₓ H, gegebenenfalls hydroxyliertes (C₁-C₁₄)-Alkyl und R_{y} hydroxyliertes (C₁-C₁₄)-Alkyl oder den Rest eines gegebenenfalls monofunktionalisierten Monosaccharids oder Oligosaccharids bedeuten, wobei R_{y} gegebenenfalls zusätzlich (C₁-C₄)-Alkylen- oder Phenylen-Gruppen aufweist, die an die oben angegebenen Gruppen über Amidfunktionen oder Etheroxidgruppen gebunden sind,
n 2 oder 3 darstellt,
und Z NRₓR_{y} oder OH bedeutet,
sowie deren Säuresalze mit anorganischen oder organischen Basen.

6. Derivate nach Anspruch 1 der Formel 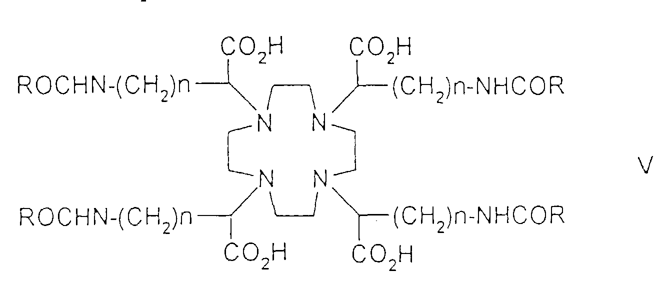 in der
n 2 oder 3 bedeutet
und R den Rest eines gegebenenfalls monofunktionalisierten Saccharids oder Oligosaccharids darstellt, und deren Säuresalze mit anorganischen oder organischen Basen.

7. Derivate nach den Ansprüchen 1 oder 2 der Formel 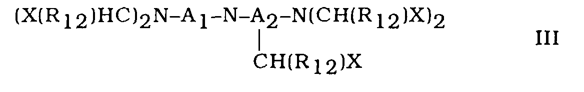 in der
- A₁ und A₂ unabhängig voneinander 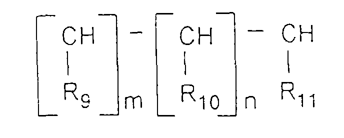 bedeuten,
worin m und n 0, 1 oder 2 darstellen und deren Summe 1 oder 2 beträgt, R₉, R₁₀ und R₁₁ unabhängig voneinander H, Alkyl, Alkoxyalkyl, Phenyl oder Alkylenphenyl darstellen, wobei R₁₀ zusätzlich OH oder Alkoxy bedeuten kann oder eine der Gruppen R₉ und R₁₁ die Formel 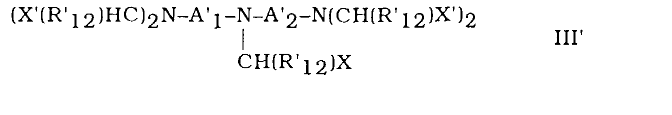 bedeutet,
in der die Buchstaben die Bedeutungen der Buchstaben des gleichen Indexes der Formel III besitzen können mit Ausnahme von R'₉ und R'₁₁, die nicht III' bedeuten können und von denen der eine Rest (C₁-C₈)-Alkylen, welches gegebenenfalls eine oder mehrere Alkoxygruppen trägt, darstellt,
R₁₂ H, Alkyl, Alkoxyalkyl oder R₁ bedeutet,
wobei die Phenylreste durch eine oder mehrere Gruppen OH, Cl, Br, I, (C₁-C₈)-Alkyl, (C₁-C₈)-Alkoxy, NO₂, NRₓR_{y}, NRₓCOR_{y}, CONRₓR_{y} oder COORₓ substituiert sein können, worin Rₓ und R_{y} H oder (C₁-C₈)-Alkyl bedeuten und die Alkyl-, Alkylen- und Alkoxyreste geradkettige oder verzweigte C₁-C₁₄-Reste sind, die gegebenenfalls hydroxyliert sind,
und deren Salze mit anorganischen oder organischen Säuren oder Basen.

8. Derivat der Formel 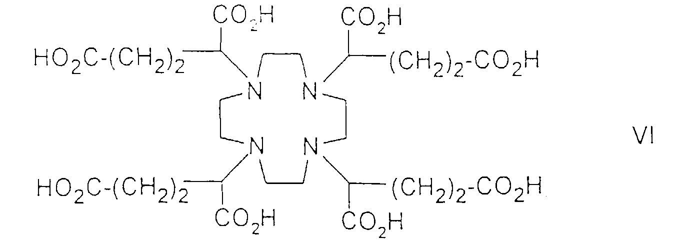 und dessen Chelate mit Metallkationen und dessen Salze mit organischen oder anorganischen Basen.

9. Derivat der Formel 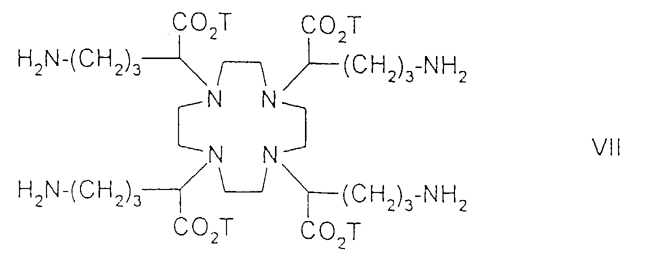 in der T H oder (C₁-C₈)-Alkyl darstellt, und dessen Chelate mit Metallkationen und dessen Salze mit organischen oder anorganischen Säuren.

10. Chelat gebildet zwischen einem paramagnetischen Metallion und einem Derivat nach einem der Ansprüche 1 bis 9.

11. Chelat nach Anspruch 10, worin das Ion das Ion von Gadolinium oder Mangan ist.

12. Zubereitung für die medizinische Bilderzeugung durch kernmagnetische Resonanz, dadurch gekennzeichnet, daß sie ein Chelat nach einem der Ansprüche 10 oder 11 und ein physiologisch verträgliches Trägermaterial umfaßt.

13. Verfahren zur Herstellung von Chelaten des Derivats der Formel IV nach Anspruch 5, welches darin besteht, auf das Chelat des Derivats der Formel 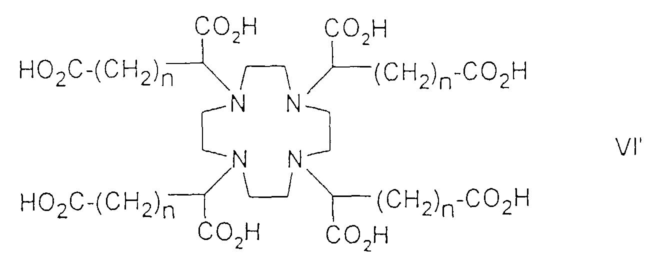 worin n 2 oder 3 bedeutet,
ein Amin der Formel NRₓR_{y}, in der Rₓ und R_{y} die in Anspruch 5 angegebenen Bedeutungen besitzen, einwirken zu lassen.

## Claims

1. Derivatives of polyamino acids, chelating agents of paramagnetic metallic ions, of formula I 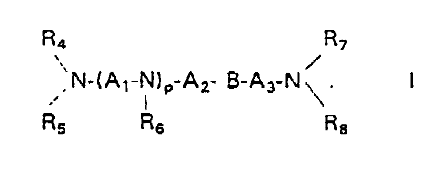 or of formula II 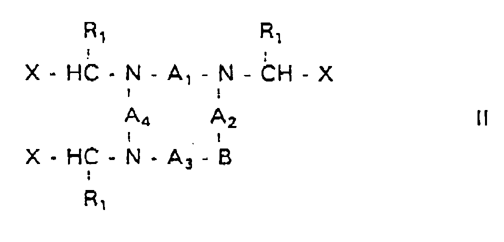 in which
• A₁, A₂, A₃ and A₄, the same or different, represent 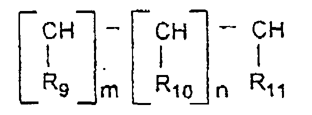
m and n being integers from 0 to 5, the sum of which equals 1 to 5 in formula I, and integers from 0 to 2, the sum of which equals 1 or 2 in formula II,
R₉, R₁₀ and R₁₁ represent independently H, alkyl, alkoxyalkyl, phenyl, alkylene phenyl, R₁₀ being able to represent also OH or alkoxy or one of the R₉'s and R₁₁ of the formula I represents 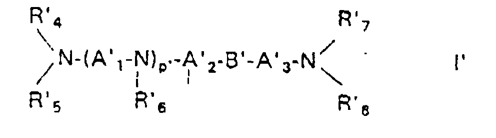 in which the letters can have the meanings of the letters of the same index of formula I, with the exception of one of the R'₉'s and R'₁₁ which represents (C₁-C₈)alkylene, possibly substituted by one or more (C₁-C₈)alkoxy, the other not being I'
or one of the R₉'s and R₁₁ of formula II represents 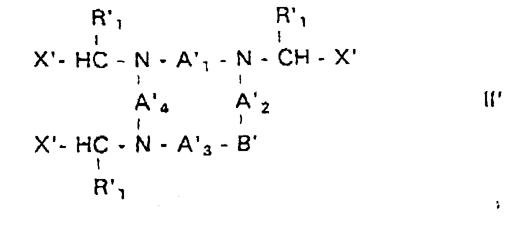 in which the letters can have the meanings of the letters of the same index of formula II, with the exception of R'₉ or R'₁₁ which is bonded to the macrocycle II and represents (C₁-C₈)akylene, possibly substituted by one or more alkoxy,
• R₄, R₅, R₆, R₇ and R₈ represent, independently, H, alkyl, alkoxyalkyl, amidoalkyl, which is possibly substituted by alkyl or alkoxyalkyl, or CH(R₁₂)X, R₁₂ being H, alkyl, alkoxyalkyl or R₁,
or the groups R₄ and R₇ are bonded and taken together represent 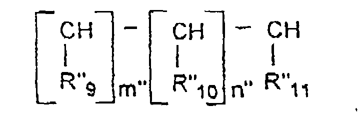 R"₉, R"₁₀, R"₁₁, m" and n" being able to take the meanings of the letters of the same index in formula I.
• B represents NCH(R₁)X or N-W and W represents H, alkyl, alkoxyalkyl, amidoalkyl, which is possibly substituted by alkyl or alkoxyalkyl, these groups possibly comprising furthermore a phenyl in the case of formula II, (C₁-C₆)alkylene-Y or Y, Y being a saturated or non-saturated heterocyclic compound, of 1 or 2 attached cycles, which is possibly substituted by OH, alkyl, alkoxy, alkoxyalkyl, having up to 12 vertices, comprising 1 to 4 heteroatoms chosen from O, N, S, it being understood that when W represents Y, the carbon bonded to N is bonded to 2 carbon atoms of the heterocyclic compound,
or W represents the group I" in formula I: 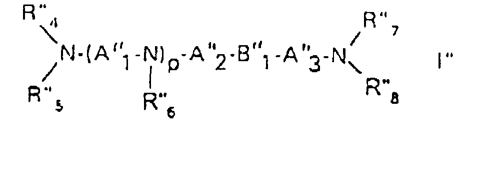 in which the letters can have the meanings of the letters of the same index of formula I with the exception of one of the R"₉ and R"₁₁ groups which cannot represent I' and of B"₁ which represents N-Q, Q being (C₁-C₈)alkylene, which is possibly substituted by one or several alkoxy, or W represents the group II' in formula II in which the letters can have the meanings of the letters of the same index in formula II with the exception of B'₂ which represents N-(C₁-C₈)alkylene, which is possibly substituted by one or several alkoxy,
or B is 0 in formula I
or A₂-B-AX₃ represents a heterocyclic group in which B is a saturated or non-saturated heterocyclic compound, of 5 to 6 vertices comprising 1 or 2 heteroatoms which are chosen from O, S, N and A₂ and A₃ represent a CH-Rₑ group in which Rₑ is H or (C₁-C₆)alkyl,
• p is a whole number from 0 to 5;
• the X's, the same or different, represent CO₂Rₐ, CONR_{b}R_{c} or P(R_{d})O₂H and Rₐ, R_{b}, R_{c}, the same or different, represent H or (C₁-C₈)akyl, possibly hydroxylated; R_{d} represents OH, (C₁-C₈)alkyl or (C₁-C₈)alkoxy,
• and R₁ represents a hydrophilic group of molecular mass greater than 200, comprising at least three atoms of oxygen, of formula R₂-G-R₃ in which R₂ represents nothing, alkylene, alkoxylene, polyalkoxylene, alkylene which is interrupted phenylene, phenylene or a saturated or non-saturated heterocyclic residue ;
G represents a function O, CO, OCO, COO, SO₃, OSO₂, CONR', NR'CO, NR'COO, OCONR', NR', NR'CS, CSNR', SO₂NR', NR'SO₂, NR'CSO, OCSNR', NR'CSNR', P(O)(OH)NR', NR'P(O)-(OH), in which R' is H, (C₁-C₈)alkyl or R₃ ;
R₃ represents alkyl, phenyl, alkyl, which is substituted or interrupted by one of the phenyl, alkyleneoxy, amino or amido groups, which are substituted or non-substituted by alkyl, which is possibly substituted or interrupted by one of the preceding groups or R₃ is the remainder of a compound, possibly monofunctionalised, chosen from the saccharides and the oligosaccharides, the phenyl, phenylene and heterocyclic groups being able to be substituted by OH, Cl, Br, I, (C₁-C₈)alkyl, (C₁-C₈)alkoxy, NO₂, NRₓR_{y}, NrₓCOR_{y}, CONRₓRₓ, COORₓR_{y}, and R_{y} being H or (C₁-C₈)alkyl and the alkyl, alkylene, alkoxy groups, which are at C₁ to C₁₄, linear, branched or cyclic, being able to be hydroxylated, it being understood that at least 3 of the complexing nitrogen atoms carry a CH(R₁)X group, as well as the salts of these derivatives with mineral or organic bases.

2. Derivatives according to claim 1, characterised in that X represents CO₂H, possibly made into a salt, and R₁ represents R₂-G-R₃ and R₂ represents (C₁-C₅)alkylene, possibly interrupted by phenylene, G represents CONR', NR'CO or O, R' being H, (C₁-C₈)alkyl or R₃, and R₃ represents (C₁-C₁₄)alkyl, possibly substituted or interrupted by one or more of the phenyl, (C₁-C₆)alkoxy, alkyleneoxy, amino and amido groups, which are substituted or non-substituted by alkyl which is possibly substituted or interrupted as previously, or the remainder of a saccharide or of an oligosaccharide, possibly monofunctionalised.

3. Derivatives according to one of the claims 1 or 2, characterised in that the groups CH(R₁)X are identical.

4. Derivatives according to claim 1 of formula II,
in which A₁, A₂, A₃ and A₄ represent (CH₂)ₙ with n - 2, 3 or one of them represents (CH₂)ₙ-CH-R₁₁ and the others (CH₂)ₙ with n' = n - 1 and R₁₁ represents alkyl, phenyl or alkylene phenyl, which are possibly substituted, the groups CH(R₁)X are identical and X is CO₂H and B represents N-W.

5. Derivatives according to claim 1 of formula 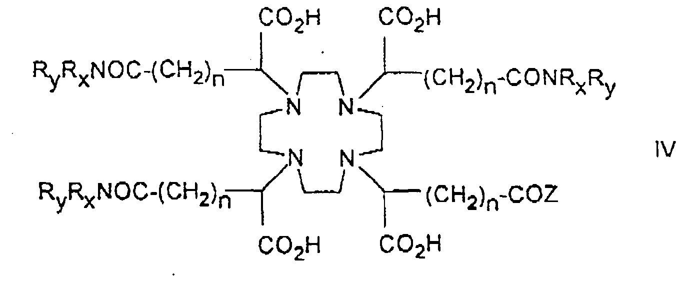 in which
Rₓ is H, (C₁-C₁₄)alkyl, possibly hydroxylated, and R_{y} is hydroxylated (C₁-C₁₄)alkyl, or the remainder of a monosaccharide or of an oligosaccharide, possibly monofunctionalised, R_{y} comprising possibly, furthermore, (C₁-C₄)alkylene or phenylene groups which are bonded to the former by amide functions or ether oxides,
n is 2 or 3,
and Z represents NRₓR_{y} or OH,
as well as their acid salts with mineral or organic bases.

6. Derivatives according to claim 1 of formula 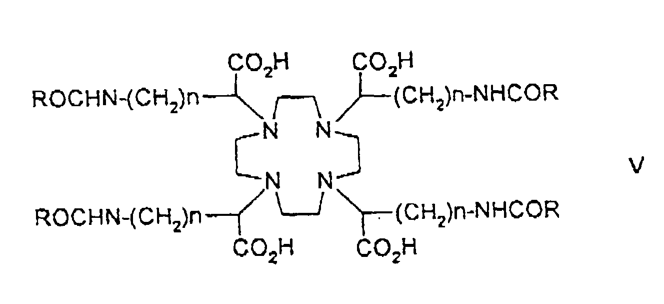 in which
n is 2 or 3,
and R represents a remainder of saccharide or of oligosaccharide, possibly monofunctionalised, and their acid salts or mineral or organic bases.

7. Derivatives according to one of the claims 1 or 2 of formula 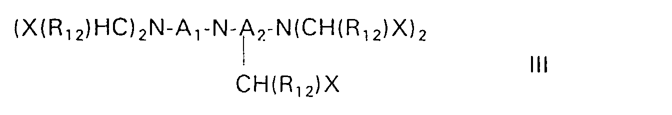 in which
• A₁ and A₂ represent independently 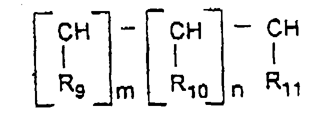
m and n being 0, 1 or 2 and their sum equalling 1 or 2,
R₉, R₁₀, R₁₁ representing independently H, alkyl, alkoxyalky, phenyl, alkylene phenyl, R₁₀ being able to represent furthermore OH or alkoxy or one of the R₉'s and R₁₁ represents the formula 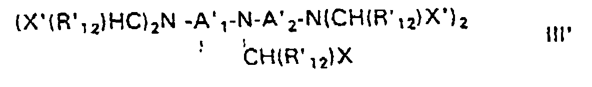 in which the letters can have the meanings of the letters of the same index of formula III with the exception of R'₉ and R'₁₁, which cannot represent III' and one of which represents (C₁-C₈)alkylene carrying possibly one or more alkoxy,
R₁₂ represents H, alkyl, alkoxyalkyl or R₁,
the phenyls being able to be substituted by one or more of the groups OH, Cl, Br, I, (C₁-C₈)alkyl, (C₁-C₈)alkoxy, NO₂, NRₓR_{y}, NRₓCOR_{y}, CONRₓR_{y}, COORₓ with RₓR_{y} being H or (C₁-C₈)alkyl and the alkyl, alkylene, alkoxy being linear or branched at C₁ to C₁₄ and possibly hydroxylated,
and their salts with acids or mineral or organic bases.

8. Derivative of the formula 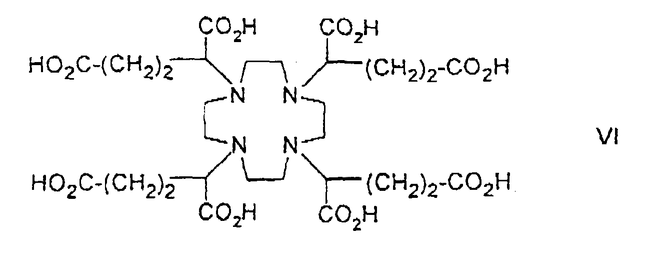 and its chelates with metallic cations and their salts with organic or mineral bases.

9. Derivative of the formula 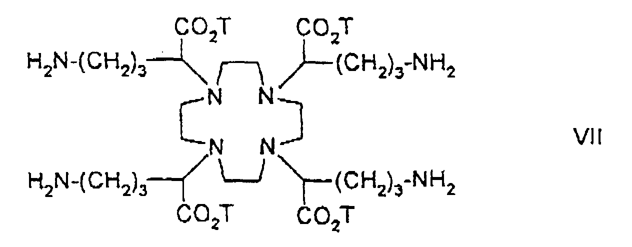 in which T represents H or (C₁-C₈)alkyl and its chelates with metallic cations and their salts with organic or mineral acids.

10. Chelate formed between a paramagnetic metallic ion and a derivative according to one of the claims 1 to 9.

11. Chelate according to claim 10, in which the ion is that of gadolinium or of manganese.

12. Compound for medical imagery by nuclear magnetic resonance, characterised in that it comprises a chelate according to one of the claims 10 or 11 and a physiologically suitable vehicle.

13. Method for the preparation of chelates of a derivative of formula IV according to claim 5, which comprises having an amine of formula NRₓR_{y}, in which Rₓ and R_{y} are as defined in claim 5, react upon the chelate of the derivative of formula 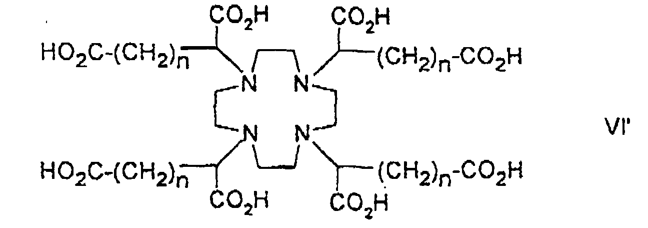 where n is 2 or 3.
